(19) Europäisches Patentamt
European Patent Office
Office européen des brevets

(11) Publication number: 0 333 071 A2

(12) # EUROPEAN PATENT APPLICATION

(21) Application number: 89104302.8

(51) Int. Cl.4: C07K 7/06 , A61K 37/02

(22) Date of filing: 10.03.89

Claim for the following Contracting State: ES

(30) Priority: 11.03.88 JP 57985/88

(43) Date of publication of application:
20.09.89 Bulletin 89/38

(84) Designated Contracting States:
AT BE CH DE ES FR GB IT LI LU NL SE

(71) Applicant: Eisai Co., Ltd.
6-10, Koishikawa 4-chome Bunkyo-ku
Tokyo 112(JP)

(72) Inventor: Tsuchiya, Yutaka
2455B, Leinghton St.
Fort Lee New Jersey, 07024(US)
Inventor: Sasaki, Atsushi
Shizanryo, 19-13, Kasuga 4-chome
Tsukuba-shi Ibaraki(JP)
Inventor: Yoshino, Hiroshi
302, 3-18, Tsukushino
Abiko-shi Chiba(JP)
Inventor: Karibe, Norio
Shizanryo, 19-13, Kasuga 4-chome
Tsukuba-shi Ibaraki(JP)
Inventor: Sugimoto, Hachiro
3073-13, Kashiwadamachi
Ushiku-shi Ibaraki(JP)
Inventor: Kubota, Atsuhiko
1130-9, Ohaza Nagakuni
Tsuchiura-shi Ibaraki(JP)
Inventor: Kosasa, Michiko
Villa Espowar 206, 14-5, Agatsuma 4-chome
Tsukuba-shi Ibaraki(JP)
Inventor: Araki, Shin
2-22, Kumauchicho 5-chome Chuo-ku
Kobe-shi Hyogo(JP)
Inventor: Ikeda, Masuhiro
6-6, Toukoudai 3-chome
Tsukuba-shi Ibaraki(JP)
Inventor: Yamanishi, Yoshiharu
2-4, Matsuba 3-chome
Ryugasaki-shi Ibaraki(JP)
Inventor: Machida, Ryoichi
94-25, Masuo
Kashiwa-shi Chiba(JP)
Inventor: Yamatsu, Kiyomi
23-7, Imaizumidai 7-chome
Kamakura-shi Kanagawa(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **Polypeptides, methods for their preparation, pharmaceutical compositions comprising them and use.**

(57) A polypeptide is shown with the following formula:
A-B-C-D-E-F-$R^1$
wherein A, B, C, D, E, F and $R^1$ are as defined in claim 1.

The invention includes pharmaceutical compositions comprising the above polypeptide and the use of this polypeptide. Finally, preparation methods for the new polypeptide are disclosed.

EP 0 333 071 A2

# Polypeptides, Methods for their Preparation, Pharmaceutical Compositions Comprising Them and Use

The present invention relates to a novel polypeptide having an excellent effect as a drug.

[Prior Art and Background of the Invention]

Major tranquillizers which are a blocker against a receptor for dopamine which is one of neurotransmitters have been used as a remedy for schizopherenia for a long time and are now used in the treatment of sequela of cerebrovascular diseases, derangement due to injury of head or senile dementia or senile insanity.

However, the major tranquillizers according to the prior art are clinically problematic in that they cause an extrapyramidal disease as an adverse reaction.

In 1973, neurotensin which is an intrinsic neuropeptide represented by the following structural formula and exhibiting a central nervous system depressant action similar to that of the major tranquillizers has been found (see J. Biol. Chem., 24, 6824 to 6861 (1973)).

P-Glu(1)-Leu(2)-Tyr(3)-Glu(4)-Asn(5)-Lys(6)-Pro(7)-Arg(8)-Arg(9)-Pro(10)-Tyr(11)-Ile(12)-Leu(13)-OH

Although this neurotensin exhibits a central nervous system depressant action similar to that of the major tranquillizers, for example, a depressant action against the enhancement in the spontaneous motion of a rat due to the administration of methamphethamine which is an indirect activator for dopamine nervous system, i.e., methamphethamine antagonism, it does not act as a blocker against dopamine nervous system and has only a weak effect upon nigrostriatal dopamine nurvous system which has a close relation to extrapyramidal diseases. Therefore, it is expected that neurotensin will be effectively used as an antipsychotic drug accompanied with a lowered extrapyramidal adverse reaction (see Trends in Pharmacological Science, 201 (1985)).

Further, neurotensin has a high analgetic effect and this effect is not hindered by naloxone. Therefore, it is also suggested that it will be effectively used as a non-narcotic analgetic.

However, neurotensin is significantly problematic in that it is unstable in vivo, so that it dose not exhibit effects as described above by general administration such as intravenous, intramuscular or subcutaneous injection or oral administration.

For the purpose of finding a derivative which can exhibit a central nervous system depressant action even by intravenous, intramuscular or subcutaneous injection or oral administration, the inventors of the present invention have made various studies over a long period of time mainly upon the chemical modification of the C-terminal fragment of neurotensin which is a minimum active fragment thereof.

As a result, they have found that a novel peptide which will be described hereinbelow can attain the above purpose. The present invention has been accomplished on the basis of this finding.

Thus, an object of the present invention is to provide a novel polypeptide useful as a drug, a process for the preparation thereof and a drug containing it as an active ingredient.

[ Summary of the Invention ]

The objective compound of the present invention is a polypeptide represented by the general formula (I) and a pharmacologically acceptable salt thereof:

A-B-C-D-E-F-$R^1$ (I)

wherein A stands for a group

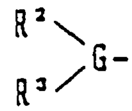

(G stands for a basic amino acid of L- or D-form, and $R^2$ and $R^3$, which may be the same or different, each stands for a hydrogen atom, a lower alkyl group or an acyl group), a group

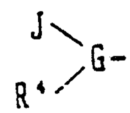

(in which G stands for a basic amino acid of L- or D-form, J stands for an amino acid of L- or D form, and $R^4$ stands for a hydrogen atom or a lower alkyl group), an ω-aminoalkanoyl group

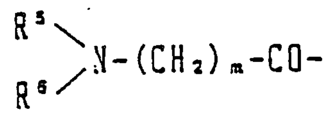

(in which $R^5$ and $R^6$, which may be the same or different, each stand for a hydrogen atom or a lower alkyl group, and m is an integer of from 1 to 10), or an ω-guanidinoalkanoyl group

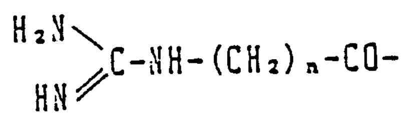

(in which n is an integer of from 1 to 10), or a group having the formula

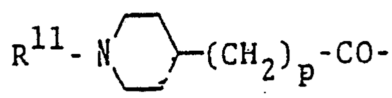

in which R11 is hydrogen or a lower alkyl, p is zero or an integer of from 1 to 10, B is a basic amino acid of L-form or an α-N-alkyl derivative thereof, C stands for L-Pro or a derivative thereof, D stands for a natural or non-natural aromatic amino acid of L-form, E stands for an amino acid of L-form or an α-N-alkyl derivative or α-C-alkyl derivative thereof, F stands for an amino acid of L- or D-form or an α-N-alkyl derivative or α-C-alkyl derivative thereof, and $R^1$ stands for a group

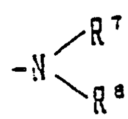

(in which $R^7$ and $R^8$, which may be the same or different, each stand for a hydrogen atom or a lower alkyl group) or a group -O-$R^9$ (in which $R^9$ stands for a hydrogen atom, an alkyl group, an alkenyl group or a group $-(CH_2)_p-O-R^{10}$ in which p is an integer of from 1 to 5 and $R^{10}$ stands for an alkyl group, an alkenyl group or an acyl group).

The invention provides a pharmaceutical composition which comprises a therapeutically effective amount of the polypeptide as defined above or a pharmacologically acceptable salt thereof and a pharmacologically acceptable carrier. The composition is useful for the methamphetamine antagonistic activity, an antipsychotic agent, the improvement and for treatment of a mental symptom associated with organic disorder in the brain, the improvement and for treatment of a mental symptom associated with a

cerebrovascular desease, the improvement and for treatment of a mental symptom associated with a penile dementia and an analogesic agent.

The above composition is useful for improving and/or treating a patient afflicted with an organic disorder in the brain, whereby to said patient a therapeutically effective amount of the composition as defined above is administered. A cerebrovascular disease and a senile dementia can be treated.

The invention further provides the use of the polypeptide as defined above for the manufacture of a medicament for the treatment of a mental symptom by an organic disorder in the brain. It extends also to a medicament as an antipsycholotic agent and a medicament as an analgesic agent.

The invention provides a process for producing the polypetide or pharmacologically acceptable salt thereof as defined above, which comprises eliminating a protected group of the polypeptide and, if neccessary, conducting an acid addition reaction.

Although an amino acid which is a constituent of peptide is generally present in D- or L-form, the term "amino acid" used in this specification refers to the one of L-form, unless stated as D-form.

Further, abbreviations which are ordinarily used in the field of peptide chemistry or synthetic organic chemistry are used in this specification. Such abbreviations will now be listed together with other abbreviations which are often used.

An $N^{\alpha}$-alkyl amino acid is represented by the abbreviation corresponding to the amino acid residue with "(alkyl)" prefixed thereto.

Example: ( $N^{\alpha}$-methylarginine ; (Me)Arg
$N^{\alpha}$-methylisoleucine ; (Me)Ile )

Tyr ; tyrosine
Tyr(Et) ; O-ethyltyrosine
Tyr(Me) ; O-methyltyrosine
Phe ; phenylalanine
Arg ; arginine
Leu ; leucine
Ile ; isoleucine
Nle : norleucine
Ser : serine
Val : valine
Nva : norvaline
homoArg ; homoarginine
Orn ; ornithine
Pro(s) ; 4-thioproline
Trp ; tryptophan
Pro(4-OH) ; 4-hydroxyproline
$\Delta^3$Pro ; 3,4-dehydroproline
Ala ; alanine
cHgl ; 2-cyclohexylglycine
Pro ; proline
Phe(p-Cl) ; p-chlorophenylalanine
Phe(p-$NO_2$) ; p-nitrophenylalanine
Tle ; tert-leucine
Leu($\gamma$-Me) ; $\gamma$-methylleucine
Leu($\alpha$-Me) ; $\alpha$-methylleucine
Trp($\alpha$-Me) ; $\alpha$-methyltryptophan
$\Delta^2$Tyr ; 2,3-dehydrotyrosine
Tyr($\alpha$-Me) ; $\alpha$-methyltyrosine
Bal ; 3-(3-benzo[b]thienyl)alanine
Nal ; 3-(1-naphthyl)alanine
Pgl ; phenylglycine
Aib ; 2-aminoisobutanoic acid
Gb ; $\omega$-guanidinobutanoic acid
Gp ; $\omega$-guanidinopentanoic acid

Gh ; ω-guanidinohexanoic acid
Pgl(p-OH) ; p-hydroxyphenylglycine
cLeu ; cycloleucine
Ge ; ω-guanidinoheptanoic acid
GABA ; γ-aminobutanoic acid
Ap ; ω-aminopentanoic acid
Ah ; ω-aminohexanoic acid
Ae ; ω-aminoheptanoic acid
Ao ; ω-aminooctanoic acid
Inp ; isonipecotic acid
Paa ; 4-piperidineacetic acid
Ac ; acetyl
Bz ; benzoyl

The lower alkyl group defined above with respect to $R^2$, $R^3$, $R^4$, $R^5$, $R^6$, $R^7$, $R^8$ and $R^{11}$ in the formula (1) is a straight-chain, branched or cyclic alkyl group having 1 to 6 carbon atoms and examples thereof include methyl, ethyl, propyl, isopropyl, butyl, isobutyl, sec-butyl, tert-butyl, pentyl, (amyl), isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclohexyl, cyclopentyl, cyclopentylmethyl and cyclohexylmethyl groups.

The alkenyl group defined with respect to $R^9$ and $R^{10}$ is any of the above-described alkyl groups which has a double bond in its carbon chain.

The alkyl group defined with respect to $R^9$ and $R^{10}$ includes not only those as defined above with respect to the lower alkyl group but also straight-chain, branched, cyclic and cyclic structure-containing long-chain alkyl groups. Particular examples of the long-chain alkyl group include n-heptyl, n-octyl, n-dodecanyl and n-octadecanyl groups.

The acyl group defined with respect to $R^2$, $R^3$ and $R^{10}$ includes acetyl, hydroxyacetyl, n-propanoyl, n-butyroyl, isobutyroyl, benzoyl, ring-substituted benzoyl, nicotinoyl, isonicotinoyl, methyloxalyl, 1-naphthoyl and 2-naphthoyl groups.

Further, the term "alkyl" in the "α-N-alkyl derivative" defined with respect to the amino acids has the same meaning as that of the "lower alkyl" defined above.

In the compound (I) of the present invention, the ω-aminoalkanoyl or ω-guanidinoalkanoyl group defined with respect to A may be any one wherein m and n are each 1 to 10. Particularly preferred examples of the former include ω-aminopentanoyl, ω-aminohexanoyl, ω-aminoheptanoyl and ω-aminooctanoyl groups which are corresponding to the cases wherein m is 4, 5, 6, 7 and 8, respectively, while preferred examples of the latter include ω-guanidinobutyroyl and ω-guanidinopentanoyl groups which are corresponding to the cases wherein n is 3 and 4, respectively.

When A is the group having the formula:

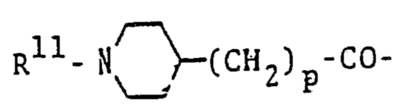

in which R11 is hydrogen or a lower alkyl and p is zero or an integer of from 1 to 10, it is preferred that R11 is hydrogen and p is zero or 1, that is to say, A is isonicopenoyl (Inp) or 4-piperidinylmethylcarbonyl (Paa).

Although the basic amino acid of L- or D-form defined with respect to G may be any one, preferred examples thereof include L-Lys, D-Lys, L-Arg, D-Arg, L-Orn, D-Orn, L-homoArg and D-homoArg.

Although the basic amino acid of L-form defined with respect to B may be any one, preferred examples thereof include L-Lys, L-Arg, L-homoArg and L-Orn.

The L-proline derivative defined with respect to G may be any one and preferred examples thereof include L-Pro, L-Pro(4-OH), L-Pro(s), L-1,3-thiazoline-2-carboxylic acid, L-3,4-dehydroproline and L-indoline-2-carboxylic acid.

Although the aromatic amino acid of L-form defined with respect to D may be any one, preferred examples thereof include L-Tyr, L-Trp, L-Phe, L-Pgl, L-Bal, L-Nal, 3-(2-benzo[bjthienyl)-L-alanine and 3-(2-naphthyl)-L-alanine.

With respect to the "O-acyl or O-alkyl derivative of Tyr" in the definition of D, preferred examples of the

acyl group include acetyl, n-propanoyl, n-butyroyl, isobutyroyl, valeroyl, benzoyl, p-toluoyl, m-toluoyl, o-toluoyl, phenylacetyl, m-methylphenylacetyl and o-methoxyphenylacetyl, while those of the alkyl group include methyl, ethyl, n-propyl, isopropyl, n-butyl, isobutyl and 1-methylpropyl groups.

With respect to the "ring-substituted L-Phe", preferred examples of the substituent include nitro, halogens such as chlorine, bromine, iodine and fluorine; trifluoromethyl, lower alkyl groups and lower alkoxy groups.

Preferred examples of the amino acid of L-form defined with respect to E include L-Val, L-Leu, L-Ile, L-Nle, L-Nva, L-Phe, L-Tle, L-α-Me-Leu, L-Pgl and L-Pgl(p-OH).

Although the amino acid of L- or D-form defined with respect to F or J may be any one, preferred examples thereof include those represented by the following abbreviations:
Gly, Ala, Val, Leu, Ile, Ser, Thr, Cys, Met, Asn, Gln, Pro, Nle, Nva, Tle, Phe

Although the foregoing description has been given to the definition of A, B, C, D, E, F and $R^1$, preferred examples of the combination thereof will now be described.

That is, preferred results are obtained, when A is a group as defined above; B is L-Lys, L-Arg, L-Orn or L-homoArg; C is L-Pro or a derivative thereof, D is an amino acid selected from the group consisting of L-Trp, L-Nal, L-Tyr and L-Bal; E is L-Tle or L-Pgl; F is L-Leu and $R^1$ is a hydroxy or alkoxy group.

A pharmacologically acceptable salt to use for the peptide of the present invention includes inorganic acid addition salts such as hydrochride, sulfate hydrobromide, perchlorate and hydroiodide and then organic acid addition salts such as acetate, oxalate, maleate, fumarate, succinate and methanesulfonate.

Typical examples of the compounds of the invention are listed in the form free of salts.

H-(Tos)Arg-Arg-Pro-Tyr-Ile-Leu-OH
H-D-(Tos)Arg-Arg-Pro-Tyr-Ile-Leu-OH
H-D-Arg-Arg-Pro-Tyr-Ile-Leu-$NH_2$
H-D-Arg-Arg-Pro-Tyr-Ile-Leu-OEt
Ac-D-Arg-Arg-Pro-Tyr-Ile-Leu-$NH_2$
Ac-D-Arg-Arg-Pro-Tyr-Ile-Leu-OEt
H-D-Lys-Arg-Pro-Tyr-Ile-Leu-OH
Gb-Arg-Pro-Tyr-Ile-Leu-OH
Arg-Arg-D-Pro-Tyr-Ile-Leu-OH
Arg-Lys-Pro-Tyr-Ile-Leu-OH
H-D-Arg-Arg-Pro-Tyr-Ile-D-Leu-OH
H-D-Arg-Arg-Pro-Phe-Ile-Leu-OH
H-D-Arg-Arg-Pro-Tyr-Leu-Leu-OH
Gb-Arg-Pro-Tyr-Ile-Leu-OEt
H-D-Arg-Arg-Pro-Trp-Ile-Leu-OEt
Gb-Arg-Pro-Tyr-Ile-(Me)Leu-OH
Gb-Arg-Pro-Tyr-Ile-Ile-Leu-OEt
Gb-Arg-Pro-Pgl (p-OH)-Ile-Leu-OEt
Gb-Arg-Pro-Pgl-Ile-Leu-OEt
Ah-Arg-Pro-Trp-(Me)Leu-Leu-OEt
Gb-Arg-Pro-Trp-Nle-Leu-OEt
Gb-Arg-Pro-Trp-Ile-Leu-OEt
Gb-Arg-Pro-Trp-Pgl-Leu-OEt
Ah-Arg-Pro-Trp-Pgl-Leu-OEt
Ah-Arg-Pro-Trp-(Me)Phe-Leu-OEt
Ah-Arg-Pro-Trp-Pgl-Phe-OEt
Ah-Arg-Pro-Trp-Ser-Leu-OEt
Gb-homoArg-Pro-Trp-Pgl-Leu-OEt
Gb-Arg-D-Pro-Trp-Pgl-Leu-OEt
Ah-Arg-Pro-Trp-Nva-Leu-OEt
Ah-Arg-Pro-(Me)Tyr-Ile-Leu-OEt
Ah-Arg-Pro(S)-Trp-Pgl-Leu-OEt
Ah-D-Arg-Pro-Trp-Pgl-Leu-OEt
Ah-Arg-Pro(OH)-Trp-Pgl-Leu-OEt
GABA-Arg-Pro-Trp-Pgl-Leu-OEt
Ao-Arg-Pro-Trp-Pgl-Leu-OEt
Ah-(Me)Arg-Pro-Trp-Pgl-Leu-OEt
Ah-Arg-Pro-Trp-Pgl-Leu-OMe

Ah-Arg-Pro-Trp-Pgl-Leu-NHEt
Ah-Arg-Pro-Trp-cLeu-Leu-OEt
Gh-Arg-Pro-Trp-Pgl-Leu-OMe
Ah-Orn-Pro-Trp-Pgl-Leu-OEt
Ac-Ah-Arg-Pro-Trp-Pgl-Leu-OEt
Gp-Arg-Pro-Trp-Pgl-Leu-OEt
H-D-Arg-Arg-Pro-Trp-Pgl-Leu-OMe
Ah-Arg-Pro-Trp-Tle-Leu-OMe
H-(Me)Arg-Arg-Pro-Trp-Pgl-Leu-OEt
Ah-Arg-Pro-Tyr-Pgl-Leu-OEt
H-D-Lys-Arg-Pro-Trp-Pgl-Leu-OEt
Ah-Arg-Pro-Trp-Pgl-Leu-$NH_2$
H-D-Arg-Lys-Pro-Trp-Pgl-Leu-OMe
H-D-Lys-Arg-Pro-Trp-Tle-Leu-OMe
H-D-Lys-Arg-Pro-Trp-Tle-Leu-$NH_2$
H-D-Lys-Arg-Pro-Trp-Tle-Leu-OH
H-D-Lys-Arg-Pro-Trp-Tle-Leu-$N(Me)_2$
H-D-Lys-Arg-Pro-Trp-Tle-Leu-OEt
H-D-Lys-Lys-Pro-Trp-Tle-Leu-OMe
H-D-Pro-D-Lys-Pro-Trp-Tle-Leu-OMe
H-(Me)Arg-Arg-Pro-Trp-Tle-Leu-OMe
Ac-D-Lys-Arg-Pro-Trp-Tle-Leu-OMe
H-D-Lys-Arg-Pro-Trp-Tle-Leu-OnHe
H-D-Lys-(Me)Arg-Pro-Trp-Tle-Leu-OMe
H-D-Lys-Arg-Pro-Trp-Aib-Leu-OEt
H-D-Lys-Arg-Pro-Tyr-Phe(p-$NO_2$)-Leu-OEt
H-D-Lys-Arg-Pro-Tyr-Tle-Leu-OEt
H-D-Lys-Arg-Pro-Nal-Tle-Leu-OEt
H-D-Lys-Arg-Pro-Trp-Leu($\alpha$-Me)-Leu-OEt
H-D-Lys-Arg-Pro-L-(Me)Trp-Tle-Leu-OEt
H-D-Lys-Arg-Pro-Trp-Tle-Leu-$O(CH_2)_{11}CH_3$
H-D-Lys-Arg-Pro-Bal-Tle-Leu-OH
H-D-Lys-Arg-Pro-$\Delta^2$Tyr-Tle-Leu-OH
H-D-Arg-Arg-Pro-Tyr-Tle-Leu-OH
H-D-Lys-Arg-Pro-Tyr-Tle-Leu-OH
H-(Me)Arg-Arg-Pro-Trp-(Me)Ile-Leu-OH
H-D-Lys-Arg-Pro-Trp-(Me)Ile-Leu-OH
Ah-Arg-Pro-Trp-(Me)Ile-Leu-OH

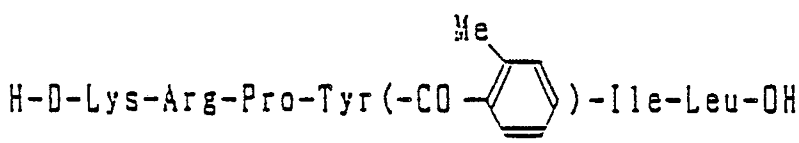

H-(Me)Arg-Arg-Pro-Trp-Tle-Leu-OH
H-D-Lys-Arg-Pro-Trp-(Me)Ile-Tle-OH

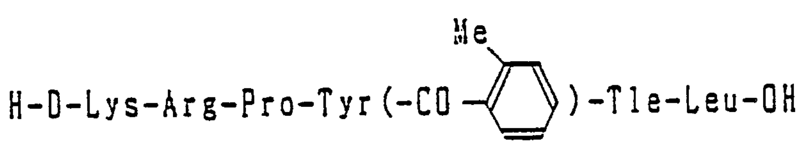

$(Me)_2$Arg-Arg-Pro-Trp-Tle-Leu-OH

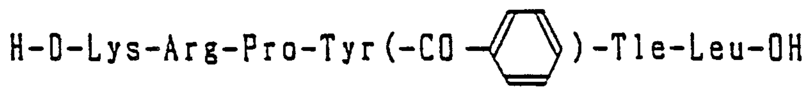

H-(Me)Arg-Lys-Pro-Tyr-Tle-Leu-OH
(Me)$_2$Arg-Lys-Pro-Tyr-Tle-Leu-OH

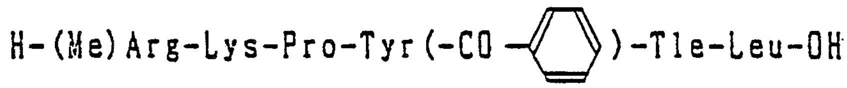

H-(Me)Lys-Arg-Pro-Tyr-Tle-Leu-OH

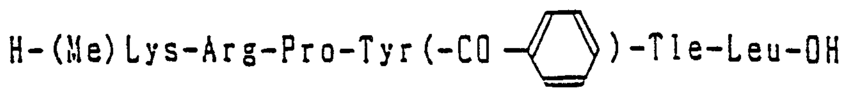

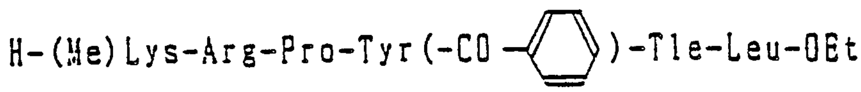

H-(Me)Lys-Arg-Pro-Trp-Tle-Leu-OH

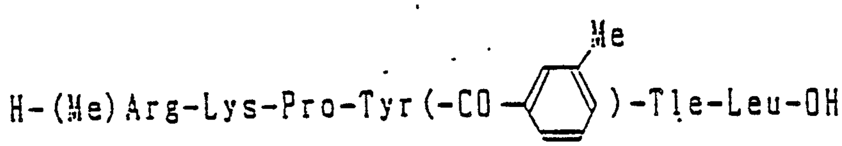

H-(Me)Arg-Lys-Pro-Tyr(-CO-CH($CH_3$)$_2$)-Tle-Leu-OH

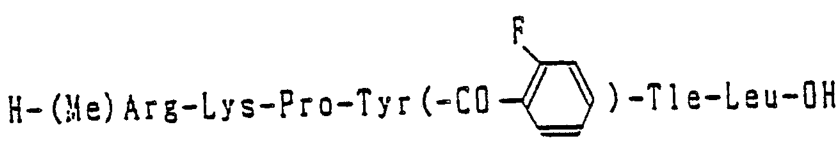

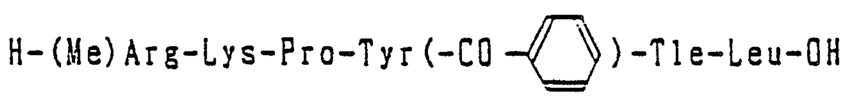

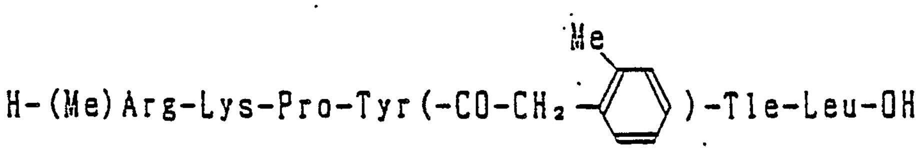

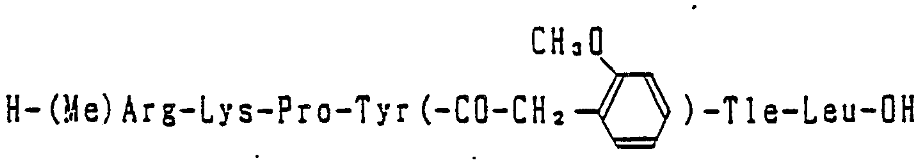

H-(Me)Arg-Lys-Pro-Trp-Tle-Leu-OH
(Me)$_2$Arg-Lys-Pro-Trp-Tle-Leu-OH
H-(Me)Arg-Lys-Pro-Trp-Tle-Leu-OEt

H-(Me)Arg-Lys-Pro-Trp-Tle-Leu-OcHe
H-(Me)Arg-Lys-Pro-Trp-Tle-Leu-OisoPr

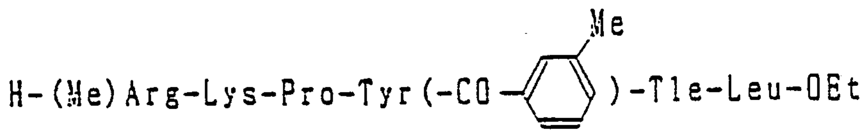

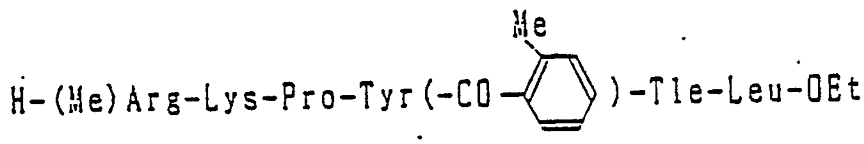

H-(Me)Arg-Lys-Pro-Tyr(-CO-CH($CH_3$)$_2$)-Tle-Leu-OEt
H-(Me)Arg-Lys-Pro-Trp-Tle-Ile-OEt
H-(Me)Arg-Lys-Pro-Trp-Tle-Leu-O($CH_2$)$_{12}$$CH_3$
H-D-Arg-Lys-Pro-Trp-Tle-Leu-OEt
H-(Me)Arg-Lys-Pro-Trp-Tle-Phe-OEt
H-(Me)Arg-Lys-Pro-Trp-Tle-Val-OEt
H-(Me)Arg-Lys-Pro-Trp-Tle-Leu-OMe
H-(Me)Arg-Lys-Pro-Trp-Tle-Leu-OisoBu
H-(Me)Arg-Lys-Pro-Trp-Tle-Leu-OnPr
H-(Me)Arg-Lys-Pro-Trp-Tle-Leu-O($CH_2$)$_{17}$$CH_3$
H-(Me)Arg-Lys-Pro-Tyr-(-Et)-Tle-Leu-OEt
H-(Me)Lys-Arg-Pro-Tyr(-Et)-Tle-Leu-OEt
H-(Me)Arg-Lys-Pro-Trp-Tle-Tle-OEt
H-(Me)Arg-Lys-Pro-Trp-Tle-Leu-O($CH_2$)$_2$OH
Ac-D-Arg-Lys-Pro-Trp-Tle-Leu-OEt
Bz-D-Arg-Lys-Pro-Trp-Tle-Leu-OEt
Ac(ME)Arg-Lys-Pro-Trp-Tle-Leu-OEt
H-(Me)Arg-Lys-Pro-Trp-Tle-Leu-OBn
H-(Me)Arg-Lys-Pro-Trp-Pro-Leu-OEt
H-(Me)Arg-Lys-Pro-Trp-Tle-D-Leu-OEt
H-(Me)Arg-Lys-Pro-Trp-Tle-Aib-OEt
H-(Me)Arg-Lys-Pro-Trp-Tle-Pro-OEt
H-(Me)Arg-Lys-Pro-Trp-Tle-Leu-O($CH_2$)$_2$OAc
H-D-Lys-Arg-Pro-Trp-Tle-Leu-O($CH_2$)$_2$OH
$CH_3$OCOCO-(Me)Arg-Lys-Pro-Trp-Tle-Leu-OEt
H-Inp-Arg-Pro-Trp-Tle-Leu-OEt
Bz-(Me)Arg-Lys-Pro-Trp-Tle-Leu-OEt
H-D-Lys-Arg-Pro-Trp-Tle-Leu-OCH$_2$OCOC($CH_3$)$_3$
H-D-Orn-Arg-Pro-Trp-Tle-Leu-OEt
H-(Me)Arg-Orn-Pro-Trp-Tle-Leu-OEt
H-D-Lys-homoArg-Pro-Trp-Tle-Leu-OEt
H-(Me)Arg-Lys-Pro-Trp-(Me)Tle-Leu-OEt
H-Paa-Arg-Pro-Trp-Tle-Leu-OEt
Ac-(Me)Arg-Lys-Pro-Trp-Tle-Leu-OH
Bz-(Me)Arg-Lys-Pro-Trp-Tle-Leu-OH
H-(Me)Arg-Lys-Pro-Trp-Tle-D-Leu-OH
H-D-Orn-Arg-Pro-Trp-Tle-Leu-OH
H-(Me)Arg-Orn-Pro-Trp-Tle-Leu-OH
H-(Me)Arg-Lys-Pro-Trp-Tle-Tle-OH
Arg-Lys-Pro-Trp-Tle-Tle-OH
Bz-D-Arg-Lys-Pro-Trp-Tle-Leu-OH
Ac-D-Arg-Lys-Pro-Trp-Tle-Leu-OH
Ac-D-Lys-Arg--Pro-Trp-Tle-Leu-OH

Gb-Arg-Pro-Trp-Pgl-Leu-OH
Ah-Arg-Pro-Trp-Pgl-Leu-OH
H-D-Lys-Arg-Pro-Nal-Tle-Leu-OH
H(Me)Orn-Arg-Pro-Trp-Tle-Leu-OH
H-D-Lys-homoArg-Pro-Trp-Tle-Leu-OH
H-Inp-Arg-Pro-Trp-Tle-Leu-OH
H-(Me)Arg-Lys-$\Delta^3$Pro-Trp-Tle-Leu-OH
H-(Me)Arg-Lys-$\Delta^3$Pro-Trp-Tle-Leu-OEt
H-D-Arg-Lys-Pro-Trp-Tle-Leu-OH

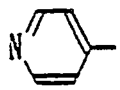 CO-D-Arg-Lys-Pro-Trp-Tle-Leu-OEt

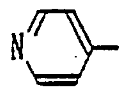 CO-(Me)Arg-Lys-Pro-Trp-Tle-Leu-OEt

HO-$CH_2$-CO-D-Arg-Lys-Pro-Trp-Tle-Leu-OEt

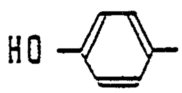 CO-D-Arg-Lys-Pro-Trp-Tle-Leu-OEt

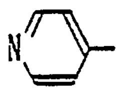 CO-D-Arg-Lys-Pro-Trp-Tle-Leu-OH

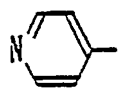 CO-(Me)Arg-Lys-Pro-Trp-Tle-Leu-OH

HO-$CH_2$-CO-D-Arg-Lys-Pro-Trp-Tle-Leu-OH

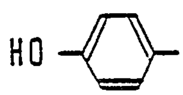 CO-D-Arg-Lys-Pro-Trp-Tle-Leu-OH

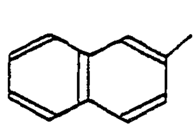 CO-D-Arg-Lys-Pro-Trp-Tle-Leu-OH

H-D-Lys-Lys-Pro-Trp-Tle-Leu-OH

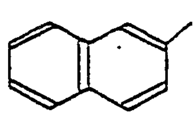 CO-D-Arg-Lys-Pro-Trp-Tle-Leu-OH

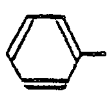 CO-D-Orn-Arg-Pro-Trp-Tle-Leu-OEt

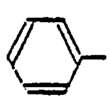 CO-D-Orn-Arg-Pro-Trp-Tle-Leu-OH

H-D-Lys-Orn-Pro-Trp-Tle-Leu-OEt

H-Inp-Lys-Pro-Trp-Tle-Leu-OEt
H-D-Orn-Lys-Pro-Trp-Tle-Leu-OEt
H-D-Inp-Orn-Pro-Trp-Tle-Leu-OEt
H-D-Orn-Orn-Pro-Trp-Tle-Leu-OEt
H-Paa-Lys-Pro-Trp-Tle-Leu-OEt
H-Paa-Orn-Pro-Trp-Tle-Leu-OEt
H-D-Lys-Orn-Pro-Trp-Tle-Leu-OH
H-Inp-Lys-Pro-Trp-Tle-Leu-OH
H-D-Orn-Lys-Pro-Trp-Tle-Leu-OH
H-Inp-Orn-Pro-Trp-Tle-Leu-OH
H-D-Orn-Orn-Pro-Trp-Tle-Leu-OH
H-Paa-Lys-Pro-Trp-Tle-Leu-OH
H-Paa-Orn-Pro-Trp-Tle-Leu-OH

According to the present invention, the significant problem of neurotensin and derivatives thereof that they cannot exhibit any central nervous system depressant action by general administration such as intravenous, intramuscular or subcutaneous injection or oral administration owing to their low stability in vivo can be solved by exerting ingenuity in the constitution of amino acids. Therefore, the compound of the present invention is highly stable in vivo and is a highly valuable one which can be put to practical use as an antipsychotic drug, a mental symptom associated and an organic disorder in the brain and then an analgetic.

The peptide of the present invention can be prepared by any suitable method. The synthesis of a protected peptide can be carried out by a conventional liquid-phase or solid-phase method. It is generally preferred to protect the pendant functional groups of the amino acids to be used and to eliminate all of the protective groups in the final step. The protective group to be used for protecting the pendant functional group of the amino acid may be any one which has already been reported and representative examples thereof include tosyl(Tos), nitro($NO_2$), benzyl(Bzl), tert-butyl($Bu^t$), benzyloxycarbonyl(Z), tert-butoxycarbonyl-(Boc) and 4-methoxy-2,3,6-trimethylbenzenesulfonyl(Mtr) groups.

Although the protective group to be used for protecting the α-amino group of the amino acid may be any one which has already been reported, it is preferable to combine a protective group for α-amino group with one for a pendant functional group so as to permit the selective elimination of the former without exerting any influence upon the latter. For example, when a tert-butoxycarbonyl group is used as the α-amino-protective group, it is preferred to use a benzyl or benzyloxycarbonyl group as the protective group for a pendant functional group, while when a benzyloxycarbonyl group is used as the α-amino-protective group, it is preferred to use a tert-butyl or tert-butoxycarbonyl group as the protective group for a pendant functional group. When the amino group of an N-terminal amino acid is dialkylated, the amino group need not be protected. It is preferred from the standpoint of inhibiting racemization during the synthesis that the synthesis of a protected peptide should be carried out by the stepwise method of binding all amino acids one by one successively from the C-terminal or by the method of fragment condensation at a position of Pro, though the latter method may be carried out in other arbitrary positions.

The peptide of the present invention can be prepared, even according to either the solid-phase or the liquid-phase method, by repeating the reaction represented by the following general reaction formula to obtain a protected peptide, eliminating the protective groups of the protected peptide and purifying the resulting peptide and the steps of the synthesis of the peptide will be each described below by taking the case of the liquid-phase method:

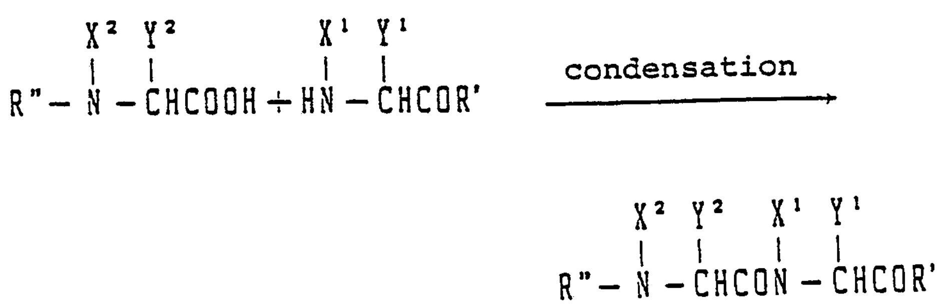

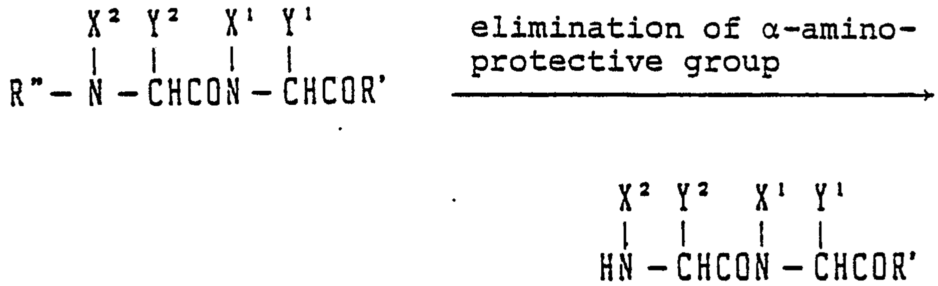


wherein $X^1$ and $X^2$ each stand for H or an alkyl group; $Y^1$ and $Y^2$ each stand for a pendant group of an amino acid and $R'$ and $R''$ each stand for a protective group or a peptide residue.

(1) Reaction for the formation of peptide linkage

The formation of peptide linkage may be carried out by any condensation method which has already been reported. Generally, the formation thereof is carried out by activating the carbonyl group of an acid component represented by the general formula:

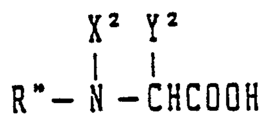

according to an ordinary method which includes the azide method, the dicyclohexylcarbodiimide (DCC) method, the mixed anhydride method or the active ester method and reacting the activated acid component with an amine component represented by the general formula:

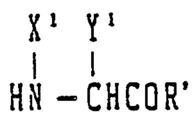

The reaction conditions (such as reaction solvent or reaction temperature) varies depending upon the method used for activating the carboxyl group. The mixed anhydride method which is one of representative condensation methods will now be described as an example. First, an acid component represented by the general formula:

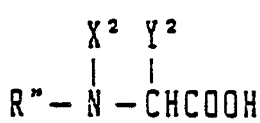

is dissolved in an aprotic solvent such as dimethylformamide, tetrahydrofuran or ethyl acetate, followed by cooling to about -20°C. N-Methylmorpholine and ethyl chlorocarbonate are added each in an equimolar amount successively. After 5 minutes, an amine component represented by the general formula:

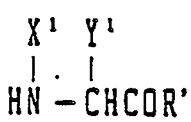

is added in an equimolar amount. The obtained mixture is stirred at -15 to 0°C for 2 to 5 hours and treated

according to an ordinary method to obtain a protected peptide represented by the general formula:

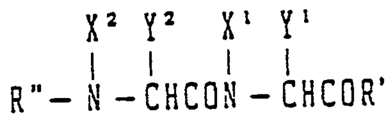

(2) Elimination of α-amino-protective group

The elimination may be carried out by an ordinary method which includes the catalytic reduction method, a method using an acid, a method using a base and a method using hydrazine. From among these methods, a suitable method is selected depending upon the kind of the α-amino-protective group to be eliminated. Representative examples of the elimination include that of a benzyloxycarbonyl group by catalytic reduction and that of a tert-butoxycarbonyl group by the use of trifluoroacetic acid. The elimination of a tert-butoxycarbonyl group by the use of trifluoroacetic acid will now be described as an example.

0.5 ml of anisole and 5 ml of trifluoroacetic acid are added to 1 g of α-N-butoxycarbonylpeptide represented by the general formula under cooling with ice:

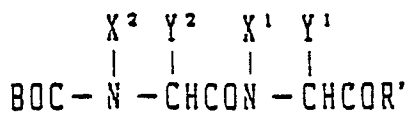

The obtained mixture is stirred for 60 minutes and treated with ether to obtain a salt of a peptide represented by the general formula:

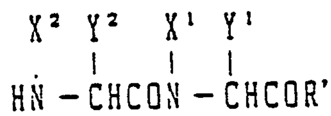

with trifluoroacetic acid. This salt is dissolved in a solvent, neutralized with an amine such as triethylamine and used in the following step.

(3) Elimination of all protective groups

The peptide chain is lengthened by repeating the above condensation and elimination of the α-amino-protective groups, followed by the elimination of all the protective groups. Thus, an objective peptide is obtained as a crude product. The elimination of all the protective groups may be carried out by an ordinary method which includes the catalytic reduction method, a method using liquid ammonia and alkali metal, a method using an acid, a method using base and a method using hydrazine. From among these methods, a suitable method is selected depending upon the kind of the protective group to be eliminated. The elimination method using hydrogen fluoride (HF) will now be described as an example of the methods which are ordinarily employed.

1 g of a protected peptide is dissolved in about 30 ml of HF in a hermetically closed HF reactor in the presence of 0.5 ml of anisole at a temperature of -15 to 0° C, followed by stirring for 60 minutes. The HF is distilled off and the obtained residue is washed with ether, dissolved in water, treated with Amberlite IRA-93 (acetic acid form) and freeze-dried to obtain a crude peptide free from any protective group.

(4) Purification of crude peptide

The purification of the crude peptide may be carried out by an ordinary method which includes ion-exchange chromatography, gel filtration, partition chromatography, countercurrent partition and high-performance liquid chromatography. The purification by high-performance liquid chromatography will now be described as an example. 100 mg of a crude peptide is fed into a column (20 ∅ x 250 mm) packed with Nucleosil 5C18 and eluted with 0.015% HCl ($H_2O$-$CH_3CN$). Fractions of objective peaks are collected by detection with UV of 210 nm and freeze-dried to obtain an objective peptide having a high purity.

The intended product can be obtained in the form of its acid addition salt, such as acetate and hydrochloride, depending on a purification method and a chemical structure of the peptide.

In order to illustrate the effect of the compound of the present invention as a drug, the results of the bioassay thereof will now be described.

Methamphethamine antagonism

This experiment was made to determine the antagonism of the compound according to the present invention against the enhancement in the quantity of spontaneous motion induced by methamphethamine.

The compounds of the present invention and physiological saline as a control were each orally or subcutaneously administered to Wister male rat (120 to 200 g, purchased from Charles River Japan Inc.) or ddY male mice (18 to 25 g, purchased from Shizuoka-ken Jikken-Dobutsu Nogyo Kyodo-Kumiai). After 30 minutes or 2 hours, methamphethamine hydrochloride (Philopon; a product of Dainippon Pharmaceutical Co., Ltd.) was administered intraperitoneally. Over a period of one hour just after this administration, the quantity of spontaneous motion was measured with MK-ANIMEX (manufactured by Muromachi Kikai).

The activity of the compound of the present invention is shown by the inhibition ratio based on the quantity of spontaneous motion of the rat or mouse to which physiological saline was administered (Table 1).

Table 1 Methamphethamine antagonism

| Test compound (Example No.) | Methamphethamine antagonism | |
|---|---|---|
| | dose (mg/kg, s.c.) | inhibition ratio (%) |
| 1 | 0.4 | 20.6 |
| 2 | 2.0 | 72.3 |
| 3 | 0.5 | 57.8 |
| 4 | 0.1 | 27.9 |
| 5 | 0.4 | 37.3 |
| 6 | 0.5 | 50.5 |
| 7 | 0.5 | 77.0 |
| 41 | 0.4 | 63.8 |
| 46 | 0.4 | 38.6 |
| 50 | 0.5 | 80.4 |
| 52 | 0.5 | 90.9 |
| 54 | 2.0 | 20.7 |
| 56 | 0.5 | 69.1 |
| 57 | 0.5 | 78.8 |
| 62 | 0.5 | 44.0 |
| 63 | 0.5 | 79.6 |

Table 1 (cont'd)

| Test compound (Example No.) | Methamphethamine antagonism | |
|---|---|---|
| | dose (mg/kg, s.c.) | inhibition ratio (%) |
| 65 | 2 | 21.6 |
| 67 | 0.5 | 51.3 |
| 71 | 2 | 68.9 |
| 72 | 0.5 | 75.1 |
| 73 | 2 | 100 |
| 74 | 0.5 | 24.2 |
| 75 | 2 | 84.2 |
| 77 | 0.5 | 97.3 |
| 78 | 0.5 | 41.3 |
| 79 | 0.5 | 39.4 |
| 80 | 0.5 | 90.1 |
| 82 | 0.5 | 94.9 |
| 83 | 0.5 | 49.3 |
| 86 | 0.5 | 69.7 |
| 87 | 0.5 | 57.2 |
| 91 | 0.5 | 18.5 |

Table 1 (cont'd)

| Test compound (Example No.) | Methamphethamine antagonism | |
|---|---|---|
| | dose (mg/kg, s.c.) | inhibition ratio (%) |
| 120 | 0.1 | 37.4 |
| 121 | 0.1 | 33.5 |
| 123 | 0.1 | 53.5 |
| 124 | 0.1 | 23.9 |
| 127 | 0.1 | 56.4 |
| 129 | 0.1 | 28.7 |
| 130 | 0.1 | 30.9 |
| 131 | 0.1 | 46.4 |
| 132 | 0.1 | 26.9 |
| 134 | 0.1 | 18.8 |
| 140 | 0.1 | 8.4 |
| 141 | 0.1 | 46.7 |
| 142 | 0.1 | 39.3 |
| 143 | 0.1 | 28.3 |
| 144 | 0.1 | 59.0 |
| 147 | 0.1 | 33.7 |
| neurotensin | >20 | — |

Table 1 (continued)

| Ex. No. | Methamphethamine antagonism | |
|---|---|---|
| | dose (mg/kg, s.c.) | inhibition ratio (%) |
| 148 | 2.0 | 61.1 |
| 151 | 2.0 | 52.2 |
| 152 | 2.0 | 47.5 |
| 153 | 2.0 | 54.4 |
| 154 | 2.0 | 55.3 |

It can be understood from the results of the pharmacological test that the activity exhibited by the general administration of the compound according to the present invention is equivalent to the one exhibited by the intraventricular administration of neurotensin, i.e., the activity of the present invention is very high.

The compound of the present invention can exhibit high methamphethamine antagonism even by general administration such as intravenous or subcutaneous injection or oral administration. Thus, the compound is highly valuable as compared with neurotensin or derivatives thereof which hardly exhibit methamphethamine antagonism by general administration because of their instability in vivo.

Further, the peptide of the present invention did not cause catalepsy which is a model symptom of an animal test on extrapyramidal diseases which are adverse reactions caused by the clinical administration of a conventional antipsychotic drug, i.e., a blocker against dopamine nervous system, even when administered in a dose which is 200 times the effective dose for methamphethamine antagonism. This fact means that the peptide has also a high clinical value.

The peptide of the present invention exhibits very high methamphethamine antagonism and analgetic effect, so that it is useful as a drug for improving and/or treating an mental symptom associated with an organic disorder in the brain, schizopherenia, senile insanity or as an analgesic. The organic disorder in the brain includes senile dementia, sequela of cerebrovascular diseases, derangement due to injury of head and so forth, from which a highly aged man suffers.

When the compound of the present invention is used as a drug, it may be administered orally or parenterally.

A drug containing the compound of the present invention as an active ingredient is prepared by converting the compound alone or together with a conventional carrier into a suitable dosage form. The carrier is selected depending upon the dosage form and examples thereof include fillers, bulk fillers, binders, moistening agent, disintegrator, surfactant and diluent such as lubricant.

The dosage form of the drug for schizopherenia according to the present invention may be selected from various forms depending upon the object of treatment and representative examples of the form include tablet, pill, powder, solution, suspension, emulsion, granule, capsule, suppository, injection (solution and suspension), ointment and inhalation, among which suppository, injection and inhalation are preferred. When the compound of the present invention is shaped into a tablet, examples of the carrier to be used include fillers such as lactose, sucrose, sodium chloride, glucose solution, urea, starch, calcium carbonate, kaolin, crystalline cellulose and silicic acid; binders such as water, ethanol, propanol, simple syrup, glucose, starch solution, gelatin solution, carboxymethylcellulose, shellac, methylcellulose, potassium phosphate and polyvinylpyrrolidone; disintegrators such as dry starch, sodium alginate, agar powder, laminaria powder, sodium hydrogencarbonate, calcium carbonate, Tween, sodium lauryl sulfate, stearic acid monoglyceride, starch and lactose; disintegration inhibitors such as sucrose, stearin, cacao butter and hydrogenated oils;

absorption accelerators such as quaternary ammonium bases, sodium lauryl sulfate, citric acid, maleic acid, fumaric acid and malic acid; humectants such as glycerin and starch; adsorbents such as starch, lactose, kaolin, bentonite and colloidal silicic acid and lubricants such as purified talc, stearates, boric acid powder, macrogol and solid polyethylene glycol. When the compound is shaped into a pill, examples of the carrier to be used include fillers such as glucose, lactose, starch, cacao butter, hardened vegetable oils, kaolin and talc; binders such as powdered acacia, tragacanth powder, gelatin and ethanol and disintegrators such as laminaria and agar. Further, if necessary, the above tablet may be coated with an ordinary material. That is, the tablet according to the present invention includes sugar coated tablets gelatin coated tablet, enteric coated tablet, film coated tablet, double-layer and multilayer tablets. When the compound is shaped into a suppository, examples of the carrier to be used include polyethylene glycol, cacao butter, higher alcohols, higher alcohol esters, gelatin and semisynthetic glyceride. The compound of the present invention may be administered by injection in the form of solution, emulsion or suspension. It is preferred that the solution, emulsion or suspension be sterilized and be isotonic to blood. In the preparation of the solution, emulsion or suspension, a diluent which has been conventionally used in this field may be used and examples of the diluent include water, ethyl alcohol, propylene glycol, ethoxylated isostearyl alcohol, polyhydroxylated isostearyl alcohol, polyoxyethylene sorbitol and sorbitan esters. Further, the drug of the present invention may contain common salt, glucose or glycerin in an amount enough to make it isotonic. Alternatively, it may contain solubilizing agent, buffer, defatting agent or preservative and, if necessary, coloring agent, preservative, correctives, sweetener and other drug.

The above drug is not particularly limited in the administration method, but may be administered by a suitable method depending upon its dosage form. For example, when the drug is in the form of tablet, pill, solution, suspension, emulsion, granule or capsule, it is administered orally, while when it is in the form of injection, it is intravenously administered as a mixture with an ordinary auxiliary liquid (such as a solution of glucose or amino acid) or is alone administered intramuscularly, intracutaneously, subcutaneously or intraperitoneally. When the drug is in the form of suppository, it is administered intrarectally, while when it is in the form of inhalation, it is administered intranasally.

When the compound of the present invention is used as an analgetic or a remedy for schizopherenia, sequela of cerebrovascular diseases, derangement due to injury of head or senile dementia or senile insanity, the dose thereof is not particularly limited, but varies depending upon the degree of symptoms, the age, sex, weight and sensitivity of a patient, the method, timing and interval of administration, the properties, preparation method and kind of a drug and the kind of an active ingredient. The compound may be generally administered by intravenous, intramuscular or subcutaneous injection in a dose of 2.5 to 1000 µg per day per adult. When the compound is orally administered, the dose thereof varies over a relatively wide range and 5 to 200 mg thereof is administered per day in several portions.

[Examples]

Examples with respect to the representative compounds of the present invention will now be described, though it is a matter of course that the present invention is not limited to them.

Example 1

Synthesis of Gb-Arg-Pro-Trp-Pgl-Leu-OEt

1) Synthesis of Boc-Pgl-Leu-OEt

8.6 g of Boc-Pgl-OH was dissolved in 100 ml of THF, followed by cooling to -20° C. 3.75 ml of N-methylmorpholine and 3.25 ml of ethyl chlorocarbonate were added to the obtained solution. After 5 minutes, a solution of 6.67 g of HCl-H-Leu-OEt and 3.75 ml of N-methylmorpholine in 50 ml of DMF was added to the obtained mixture. The resulting mixture was stirred at -50° C for 2 hours and filtered to remove precipitates. The filtrate was concentrated and the residue was dissolved in ethyl acetate to obtain a solution. This solution was washed with an aqueous solution of $NaHCO_3$ and water successively and evaporated to dryness to obtain 9.7 g of Boc-Pgl-Leu-OEt as a crystal.

∘ TLC ; Rf value = 0.41

(methanol : chloroform = 1 : 30)

- Angle of rotation ; $[\alpha]_D$ = +62.2°

(C = 0.55, dichloromethane)

- Elemental analysis ; $C_{21}H_{32}N_2O_5$

| | C | H | N |
|---|---|---|---|
| calculated (%) | 64.26 | 8.22 | 7.14 |
| found (%) | 64.13 | 8.22 | 7.22 |

2) Synthesis of Z-Trp-Pgl-Leu-OEt

1.15 g of Z-Trp-OH was dissolved in 10 ml of THF, followed by cooling to -20°C. 0.37 ml of N-methylmorpholine and 0.32 ml of ethyl chlorocarbonate were added to the obtained solution to obtain a mixture. After 5 minutes, a solution of 1.32 g of $CF_3COOH$-H-Pgl-Leu-OEt prepared by treating Boc-Pgl-Leu-OEt with $CF_3COOH$ in the presence of anisole and 0.37 ml of N-methylmorpholine in 5 ml of THF was added to the mixture. The obtained mixture was stirred at -5°C for 2 hours and filtered to remove precipitates. The filtrate was concentrated and the residue was dissolved in ethyl acetate to obtain a solution. This solution was washed with an aqueous solution of $NaHCO_3$ and water successively and evaporated to dryness to obtain 1.80 g of Z-Try-Pgl-Leu-OEt as a glassy substance.

- TLC ; Rf value = 0.61

(methanol : chloroform = 1 : 8)

- Angle of rotation ; $[\alpha]_D$ = +24.4°

(C = 0.75, dichloromethane)

- Elemental analysis ; $C_{35}H_{40}N_4O_6$

| | C | H | N |
|---|---|---|---|
| calculated (%) | 68.61 | 6.58 | 9.14 |
| found (%) | 68.45 | 6.46 | 9.08 |

3) Synthesis of Boc-Arg(Tos)-Pro-OBzl

4.28 g of Boc-Arg(Tos)-OH was dissolved in 50 ml of THF followed by cooling to -20°C. 1.1 ml of N-methylmorpholine and 0.95 ml of ethyl chlorocarbonate were added to the solution to obtain a mixture. After 5 minutes, a solution of 2.42 g of HCl-H-Pro-OBzl and 1.1 ml of N-methylmorpholine in 10 ml of DMF was added to the mixture. The obtained mixture was stirred at -5°C for 2 hours and filtered to remove precipitates. The filtrate was concentrated and the residue was dissolved in ethyl acetate to obtain a solution. This solution was washed with an aqueous solution of $NaHCO_3$ and water successively and evaporated to dryness. The residue was solidified with AcOEt-$Et_2O$ to obtain 3.75 g of Boc-Arg(Tos)- Pro-OBzl as a powder.

- TLC ; Rf value = 0.38

(methanol : chloroform = 1 : 12)

- Angle of rotation ; $[\alpha]_D$ = -28.7°

(C = 1.2, dichloromethane)

- Elemental analysis ; $C_{30}H_{41}N_5O_7S \cdot 0.5H_2O$

| | C | H | N |
|---|---|---|---|
| calculated (%) | 57.68 | 6.78 | 11.21 |
| found (%) | 57.41 | 6.58 | 11.85 |

4) Synthesis of Tos-Gb-Arg(Tos)-Pro-OBzl

3.07 g of $N^g$-Tos-γ-guanidinobutanoic acid was dissolved in 500 ml of THF, followed by cooling to -20°C. 1.13 ml of N-methylmorpholine and 0.98 ml of ethyl chlorocarbonate were added to the obtained solution to obtain a mixture. After 5 minutes, a solution of $CF_3COOH$-H-Arg(Tos)-Pro-OBzl prepared by treating Boc-Arg(Tos)-Pro-OBzl with $CF_3COOH$ in the presence of anisole and 1.13 ml of N-methylmorpholine in 20 ml of THF was added to the mixture. The obtained mixture was stirred at -5°C for 2 hours and filtered to remove precipitates. The filtrate was concentrated and the residue was dissolved in ethyl acetate to obtain a solution. This solution was washed with an aqueous solution of $NaHCO_3$ and water successively and evaporated to dryness. The obtained residue was purified by silica gel chromatography to obtain 5.2 g of Tos-Gb-Arg(Tos)-Pro-OBzl.

- TLC ; Rf value = 0.56
(methanol : chloroform = 1 : 3)
- Angle of rotation ; $[\alpha]_D$ = -31.6°
(C = 1.15, methanol : dichloromethane = 1 : 1)
- Elemental analysis; $C_{37}H_{48}N_8O_8S_2 \cdot CH_3OH \cdot H_2O$

| | C | H | N |
|---|---|---|---|
| calculated (%) | 54.11 | 6.41 | 13.29 |
| found (%) | 54.23 | 6.18 | 12.98 |

5) Synthesis of Tos-Gb-Arg(Tos)-Pro-Trp-Pgl-Leu-OEt

400 mg of Tos-Gb-Arg(Tos)-Pro-OH prepared by the catalytic reduction of Tos-Gb-Arg(Tos)-Pro-OBzl with $H_2$ gas in the presence of a 10% Pd-C catalyst and 271 mg of H-Trp-Pgl-Leu-OEt prepared by the catalytic reduction of Z-Trp-Pgl-Leu-OEt with $H_2$ gas in the presence of a 10% Pd-C catalyst were dissolved in 10 ml of a THF-DMF (10 : 1) mixture, followed by cooling to 5°C. 97.8 mg of N-hydroxysuccinimide and 117 mg of dicyclohexylcarbodiimide were added to the obtained solution successively. The obtained mixture was stirred at 5°C for 3 hours and filtered to remove precipitates. The obtained filtrate was evaporated to dryness and the residue was purified by silica gel column chromatography to obtain 380 mg of Tos-Gb-Arg(Tos)-Pro-Trp-Pgl-Leu-OEt.

- TLC ; Rf value = 0.46
(methanol : chloroform = 1 : 6)
- Angle of rotation ; $[\alpha]_D$ = -43.9°
(C = 0.40, dichloromethane)
- Elemental analysis ; $C_{57}H_{74}N_{12}O_{11}S_2 \cdot 2H_2O$

| | C | H | N |
|---|---|---|---|
| calculated (%) | 56.89 | 6.53 | 13.97 |
| found (%) | 56.97 | 6.32 | 13.97 |

6) Synthesis of Gb-Arg-Pro-Trp-Pgl-Leu-OEt

370 mg of Tos-Gb-Arg(Tos)-Pro-Trp-Pgl-Leu-OEt was dissolved in 10 ml of HF in a hermetically closed HF reactor in the presence of 1 ml of anisole at -10°C. The obtained solution was stirred for one hour and distilled to remove the HF. The residue was washed with ether and dissolved in water to obtain a solution. This solution was treated with Amberlite IRA-93 (acetic acid form) and freeze-dried to obtain a crude peptide. This crude peptide was purified with carboxymethylcellulose ion-exchanger (1.3 ∅ x 10 mm, eluted with $H_2O$-0.4 M ammonium acetate according to the gradient method) and freeze-dried to obtain 230 mg of Gb-Arg-Pro-Trp-Pgl-Leu-OEt as its acetic acid salt.

- TLC ; Rf value = 0.37

(butanol : acetic acid : water = 4 : 1 : 5)
- Angle of rotation; $[\alpha]_D$ = -47.8°
(C = 0.8, 1% acetic acid, v/v)
- Mass spectrometric analysis (FAB) ; 859 [$(M+H)^+$]
- Amino acid analysis ; Pro 1.01(1), Leu 1.01(1), Trp 0.85(1), Arg 1.13(1)

Example 2

Synthesis of Ah-Arg-Pro-Trp-Pgl-Leu-OEt

1) Synthesis of Boc-Ah-Arg(Tos)-Pro-OBzl

1.16 g of N-Boc-ω-aminocaproic acid was dissolved in 20 ml of THF, followed by cooling to -20° C. 0.55 ml of N-methylmorpholine and 0.48 ml of ethyl chlorocarbonate were added to the obtained solution to obtain a mixture. A solution of 3.12 g of $CF_3COOH$-H-Arg(Tos)-Pro-OBzl and 0.55 ml of N-methylmorpholine in 10 ml of THF was added to the mixture. The obtained mixture was stirred at -5° C for 2 hours and filtered to remove precipitates. The filtrate was evaporated to dryness and the residue was dissolved in ethyl acetate to obtain a solution. This solution was washed with an aqueous solution of $NaHCO_3$ and water successively and evaporated to dryness to obtain 3.6 g of Boc-Ah-Arg(Tos)-Pro-OBzl as a glassy substance.
- TLC ; Rf value = 0.49
(methanol : chloroform = 1 : 8)
- Angle of rotation ; $[\alpha]_D$ = -44.1°
(C = 0.77, dichloromethane)
Elemental analysis ; $C_{36}H_{52}N_6O_8S \cdot 0.3H_2O$

| | C | H | N |
|---|---|---|---|
| calculated (%) | 58.88 | 7.22 | 11.44 |
| found (%) | 58.90 | 7.08 | 11.59 |

2) Synthesis of Boc-Ah-Arg(Tos)-Pro-Trp-Pgl-Leu-OEt

534 mg of Boc-Ah-Arg(Tos)-Pro-OH prepared by the catalytic reduction of Boc-Ah-Arg(Tos)-Pro-OBzl in the presence of a 10% Pd-C catalyst was dissolved in 10 ml of a THF-DMF (10 : 1) mixture, followed by cooling to 5° C. 115 mg of N-hydroxysuccinimide and 172 mg of dicyclohexylcarbodiimide were added thereto successively. The obtained mixture was stirred at 5° C overnight, followed by the addition of a solution of 400 mg of H-Trp-Pgl-Leu-OEt in 4 ml of THF. The obtained mixture was further stirred at 5° C overnight and filtered to remove precipitates. The filtrate was concentrated and the residue was dissolved in ethyl acetate to obtain a solution. This solution was washed with an aqueous solution of $NaHCO_3$ and water and evaporated to dryness. The residue was purified by silica gel column chromatography to obtain 520 mg of Boc-Ah-Arg(Tos)-Pro-Trp-Pgl-Leu-OEt.
- TLC ; Rf value = 0.45
(methanol : chloroform = 1 : 6)
- Angle of rotation ; $[\alpha]_D$ = -37.3°
(C = 0.22, dichloromethane)
- Elemental analysis ; $C_{56}H_{78}N_{10}O_{11}S \cdot 1.5H_2O$

| | C | H | N |
|---|---|---|---|
| calculated (%)<br>found (%) | 59.72<br>59.77 | 7.25<br>7.00 | 12.44<br>12.53 |

3) Synthesis of Ah-Arg-Pro-Trp-Pgl-Leu-OEt

500 mg of Boc-Ah-Arg(Tos)-Pro-Trp-Pgl-Leu-OEt was dissolved in 10 ml of HF in a hermetically closed HF reactor in the presence of 1 ml of anisole at -10°C to obtain a solution. This solution was stirred for one hour and distilled to remove the HF. The residue was washed with ether and dissolved in water to obtain a solution. This solution was treated with Amberlite IRA-93 (acetic acid form) and freeze-dried to obtain a crude peptide. This crude peptide was purified with carboxymethylcellulose ion-exchanger (1.5 ∅ x 15 cm, $H_2O$-0.4 M ammonium acetate, the gradient method) and freeze-dried to obtain 304 mg of Ah-Arg-Pro-Trp-Pgl-Leu-OEt as its acetic acid salt.

- TLC ; Rf value = 0.55
(butanol : acetic acid : water = 4 : 1 : 1)
- Angle of rotation ; $[\alpha]_D$ = -45.3°
(C = 0.8, 1% acetic acid)
- Mass spectrometric analysis (FAB) ; 845 $[(M+H)^+]$
- Amino acid analysis ; Pro 0.96(1), Leu 1.10(1), Trp 0.88(1), Arg 1.06(1)

Example 3

Synthesis of Bz-D-Arg-Lys-Pro-Trp-Tle-Leu-OEt

1) Synthesis of Z-Tle-Leu-OEt

10.8 g of Z-Tle-OH was dissolved in 120 ml of THF, followed by cooling to -20°C. 4.46 ml of N-methylmorpholine and 3.86 ml of ethyl chlorocarbonate were added thereto successively. After 10 minutes, a solution of 7.93 g of HCl-H-Leu-OEt in 500 ml of DMF was added thereto and the obtained mixture was stirred at -5°C for 2 hours and filtered to remove precipitates. The filtrate was concentrated and the obtained residue was dissolved in ethyl acetate to obtain a solution. This solution was washed with an aqueous solution of $Na_2CO_3$ and water successively and evaporated to dryness to obtain 14.0 g of Z-Tle-Leu-OEt as an oil.

- TLC ; Rf value = 0.68
(methanol : dichloromethane = 1 : 100)
- Angle of rotation ; $[\alpha]_D$ = -8.2°
(C = 1.8, dichloromethane)
- Elemental analysis; $C_{22}H_{34}N_2O_5 \cdot 0.3H_2O$

| | C | H | N |
|---|---|---|---|
| calculated (%)<br>found (%) | 64.15<br>64.32 | 8.47<br>8.44 | 6.80<br>6.90 |

2) Synthesis of Z-Trp-Tle-Leu-OEt

13.9 g of Z-Trp-OH was dissolved in 200 ml of THF, followed by cooling to -20°C. 4.5 ml of N-methylmorpholine and 3.91 ml of ethyl chlorocarbonate were added thereto. After 5 minutes, a solution of 12.6 g of HCl-H-Tle-Leu-OEt prepared by the catalytic reduction of Z-Tle-Leu-OEt in the presence of Pd-C and 4.5 ml of N-methylmorpholine in 50 ml of THF was added thereto and the obtained mixture was stirred at -5°C for 2 hours and filtered to remove precipitates. The filtrate was concentrated and the obtained residue was dissolved in ethyl acetate to obtain a solution. This solution was washed with an aqueous solution of $Na_2CO_3$ and water and evaporated to dryness. The obtained residue was purified by silica gel column chromatography to obtain 21.3 g of Z-Trp-Tle-Leu-OEt as an amorphous substance.

◦ TLC ; Rf value = 0.44
(methanol : dichloromethane = 1 : 40)
◦ Angle of rotation ; $[\alpha]_D$ = -23.6° (C = 1, DMF)
◦ Elemental analysis ; $C_{33}H_{44}N_4O_6$

| | C | H | N |
|---|---|---|---|
| calculated (%) | 66.87 | 7.48 | 9.45 |
| found (%) | 66.74 | 7.54 | 9.52 |

3) Synthesis of Boc-Lys(Z)-Pro-OMe

3.0 g of Boc-Lys(Z)-OH was dissolved in 30 ml of THF, followed by cooling to -20°C. 0.87 ml of N-methylmorpholine and 0.75 ml of ethyl chlorocarbonate were added thereto. After 5 minutes, a solution of 1.3 g of HCl-H-Pro-OMe and 0.87 ml of N-methylmorpholine in 15 ml of a THF-DMF (1 : 1) mixture was added thereto and the obtained mixture was stirred at -5°C for 2 hours and filtered to remove precipitates. The filtrate was concentrated and the residue was dissolved in ethyl acetate to obtain a solution. This solution was washed with an aqueous solution of $Na_2CO_3$ and water successively and evaporated to dryness. The obtained residue was purified by silica gel column chromatography to obtain 35 g of Boc-Lys-(Z)-Pro-Ome.

◦ TLC ; Rf value = 0.39
(methanol : dichloromethane = 1 : 40)
◦ Angle of rotation ; $[\alpha]_D$ = -38.7°
(C = 1.7, dichloromethane)
◦ Elemental analysis ; $C_{25}H_{37}N_3O_7 \cdot 0.3H_2O$

| | C | H | N |
|---|---|---|---|
| calculated (%) | 59.98 | 7.65 | 8.39 |
| found (%) | 59.85 | 7.39 | 8.31 |

4) Synthesis of Boc-D-Arg(Tos)-Lys(Z)-Pro-OMe

4.24 g of Boc-D-Arg(Tos)-OH was dissolved in 50 ml of THF, followed by cooling to -20°C. 1.09 ml of N-methylmorpholine and 0.94 ml of ethyl chlorocarbonate were added thereto. After 5 minutes, a solution of 510 g of $CF_3COOH$-H-Lys(Z)-Pro-OMe prepared by treating Boc-Lys(Z)-Pro-OMe with $CF_3COOH$ in the presence of anisole in 30 ml of THF was added thereto. The obtained mixture was stirred at -5°C for 2 hours and filtered to remove precipitates. The filtrate was concentrated and the residue was dissolved in ethyl acetate to obtain a solution. This solution was washed with an aqueous solution of $Na_2CO_3$ and water and evaporated to dryness. The residue was purified by silica gel column chromatography to obtain 5.95 g of Boc-D-Arg(Tos)-Lys(Z)-Pro-OMe.

◦ TLC ; Rf value = 0.17
(methanol : dichloromethane = 1 : 40)
◦ Angle of rotation ; $[\alpha]_D$ = -24.2°
(C = 1.3, dichloromethane)
◦ Elemental analysis ; $C_{38}H_{55}N_7O_{10}S \cdot 1H_2O$

| | C | H | N |
|---|---|---|---|
| calculated (%) | 55.66 | 7.01 | 11.96 |
| found (%) | 55.65 | 6.77 | 11.87 |

5) Synthesis of Boc-D-Arg(Tos)-Lys(Z)-Pro-OH

5.95 g of Boc-D-Arg(Tos)-Lys(Z)-Pro-OMe was dissolved in 90 ml of methanol, followed by the addition thereto of 30 ml of N-NaOH. The obtained mixture was stirred at a room temperature for 2 hours and cooled to 5°C, followed by the addition thereto of 30 ml of N-HCl. The obtained mixture was distilled to remove the methanol. The obtained residue was acidified with N-HCl, followed by the addition thereto of ethyl acetate. The ethyl acetate layer was washed with water and evaporated to dryness to obtain 5.34 g of Boc-D-Arg-(Tos)-Lys(Z)-Pro-OH.

∘ TLC ; Rf value = 0.36
(methanol : dichloromethane = 1 : 10)
∘ Elemental analysis ; $C_{37}H_{53}N_7O_{10}S \cdot 1.5H_2O$

| | C | H | N |
|---|---|---|---|
| calculated (%) | 54.53 | 6.93 | 12.03 |
| found (%) | 54.80 | 6.78 | 12.01 |

6) Synthesis of Boc-D-Arg(Tos)-Lys(Z)-Pro-Trp-Tle-Leu-OEt

2.76 g of Boc-D-Arg(Tos)-Lys(Z)-Pro-OH and 1.60 g of H-Trp-Tle-Leu-OEt prepared by the catalytic reduction of Z-Trp-Tle-Leu-OEt in the presence of Pd-C were dissolved in 20 ml of DMF, followed by cooling to 5°C. 0.57 g of N-hydroxybenzotriazole and 0.76 g of dicyclohexylcarbodiimide were added thereto. The obtained mixture was stirred at 5°C for 3 hours and filtered to remove precipitates. The filtrate was concentrated and the residue was dissolved in ethyl acetate to obtain a solution. This solution was washed with an aqueous solution of $Na_2CO_3$ and water and evaporated to dryness to obtain 4.1 g of Boc-D-Arg(Tos)-Lys(Z)-Pro-Trp-Tle-Leu-OEt.

∘ TLC ; Rf Value = 0.61
(methanol : dichloromethane = 1 : 15)
∘ Angle of rotation ; $[\alpha]_D$ = -45.2°
(C = 0.45, dichloromethane)
∘ Elemental analysis ; $C_{62}H_{89}N_{11}O_{13}S \cdot 1.5H_2O$

| | C | H | N |
|---|---|---|---|
| calculated (%) | 59.31 | 7.39 | 12.27 |
| found (%) | 59.35 | 7.28 | 12.59 |

7) Synthesis of Bz-D-Arg(Tos)-Lys(Z)-Pro-Trp-Tle-Leu-OEt

1.2 g of HCl-H-D-Arg(Tos)-Lys(Z)-Pro-Trp-Tle-Leu-OEt prepared by treating Boc-D-Arg(Tos)-Lys(Z)-Pro-Trp-Tle-Leu-OEt with $CF_3COOH$ in the presence of anisole and converting the obtained compound with 10% HCl/ethyl acetate into its hydrochloride was dissolved in 20 ml of THF, followed by successive addition thereto of 0.25 ml of N-methylmorpholine and 0.174 g of benzoyl chloride. The obtained mixture was stirred at a room temperature for 5 hours and concentrated. The obtained residue was dissolved in ethyl acetate to obtain a solution. This solution was washed with an aqueous solution of $Na_2CO_3$ and water. The residue was purified by silica gel column chromatography to obtain 0.90 g of Bz-D-Arg(Tos)-Lys(Z)-Pro-Trp-Tle-Leu-OEt.

° TLC ; Rf value = 0.49
(methanol : dichloromethane = 1 : 15)
° Angle of rotation ; $[\alpha]_D$ = -39.9°
(C = 0.19, dichloromethane)
° Elemental analysis ; $C_{64}H_{85}N_{11}O_{12}S \cdot 4.5H_2O$

| | C | H | N |
|---|---|---|---|
| calculated (%) | 58.52 | 7.21 | 11.73 |
| found (%) | 58.36 | 6.68 | 12.15 |

8) Synthesis of Bz-D-Arg-Lys-Pro-Trp-Tle-Leu-OEt

0.90 g of Bz-D-Arg(Tos)-Lys(Z)-Pro-Trp-Tle-Leu-OEt was dissolved in 30 ml of HF in a hermetically closed HF reactor in the presence of 0.5 ml of anisole at -10° C to obtain a solution. This solution was stirred for one hour and distilled to remove the HF. The residue was washed with ether and dissolved in water to obtain a solution. This solution was treated with Amberlite IRA-93C (acetic acid form) and freeze-dried to obtain a crude peptide. This crude peptide was purified by high-performance liquid chromatography [Nucleosil-5C18, 2.25 ø x 25 cm, eluted with 0.015% HCl ($H_2O$-$CH_3CN$ = 76 : 24)] and freeze-dried to obtain 91 mg of Bz-D-Arg-Lys-Pro-Trp-Tle-Leu-OEt as its hydrochloride.

° TLC ; Rf value = 0.42
(butanol : acetic acid : water = 4 : 1 : 5)
° Angle of rotation ; $[\alpha]_D$ = -83.2°
(C = 0.75, 1% acetic acid)
° Mass spectrometric analysis (FAB) ; 944 $[(M+H)^+]$
° Amino acid analysis ; Pro 0.90(1), Leu 0.94(1), Lys 1.08(1), Trp 0.93(1), Arg 1.16(1)

Example 4

Synthesis of Bz-D-Arg-Lys-Pro-Trp-Tle-Leu-OH

30 mg of Bz-D-Arg-Lys-Pro-Trp-Tle-Leu-OEt was dissolved in 1 ml of $H_2O$, followed by the addition thereto of 0.24 ml of N-NaOH. The obtained mixture was stirred at a room temperature for 2 hours and neutralized with 0.24 ml of N-HCl. The resulting mixture was purified by high-performance liquid chromatography [Chemcosorb 5-ODS-H, 2.0 ø x 25 cm, eluted with 0.015% HCl ($H_2O$-$CH_3CN$ = 70 : 30)] and freeze-dried to obtain 12 mg of Bn-D-Arg-Lys-Pro-Trp-Tle-Leu-OH as its hydrochloride in the form of powder.

° TLC ; Rf value = 0.36
(n-butanol : acetic acid : water = 4 : 1 : 5)
° Angle of rotation ; $[\alpha]_D$ = -76.8°
(C = 0.6, 1% acetic acid)
° Mass spectrometric analysis (FAB) ; 916 $[(M+H)^+]$
° Amino acid analysis ; Pro 1.03(1), Leu 0.89(1), Lys 1.05(1), Trp 0.93(1), Arg 1.10(1)

Example 5

Synthesis of H-D-Lys-Arg-Pro-Trp-Tle-Leu-OH

1) Synthesis of Z-Tle-Leu-$OBu^t$

3.25 g of Z-Tle-OH was dissolved in 30 ml of THF, followed by cooling to -20° C. 1.34 ml of N-methylmorpholine and 1.16 ml of ethyl chlorocarbonate were added thereto successively. After 5 minutes, a solution of 2.28 g of H-Leu-OBu$^t$ in 10 ml THF was added thereto. The obtained mixture was stirred at -5° C for 2 hours and filtered to remove precipitates. The filtrate was concentrated and the residue was dissolved in ethyl acetate to obtain a solution. This solution was washed with an aqueous solution of $NaHCO_3$ and water and evaporated to dryness to obtain 5.3 g of Z-Tle-Leu-OBu$^t$.

° TLC ; Rf value = 0.54
(methanol : chloroform = 1 : 20)
° Angle of roration ; $[\alpha]_D$ = -11.6°
(C = 2.9, dichloromethane)
° Elemental analysis ; $C_{24}H_{38}N_2O_5 \cdot 0.3H_2O$

| | C | H | N |
|---|---|---|---|
| calculated (%). | 65.52 | 8.84 | 6.37 |
| found (%) | 65.31 | 8.69 | 6.22 |

2) Synthesis of Z-Trp-Tle-Leu-OBu$^t$

2.78 g of Z-Trp-OH was dissolved in 30 ml of THF, followed by cooling to -20° C. 0.91 ml of N-methylmorpholine and 0.785 ml of ethyl chlorocarbonate were successively added thereto. After 5 minutes, a solution of 2.48 g of H-Tle-Leu-OBu$^t$ prepared by the catalytic reduction of Z-Tle-Leu-OBu$^t$ in the presence of 10% Pd-C in 10 ml of THF was added thereto. The obtained mixture was stirred at -5° C for 2 hours and filtered to remove precipitates. The obtained filtrate was concentrated and the residue was dissolved in ethyl acetate to obtain a solution. This solution was washed with an aqueous solution of $NaHCO_3$ and water and evaporated to dryness to obtain 4.45 g of Z-Trp-Tle-Leu-OBu$^t$.

° TLC ; Rf value = 0.30
(methanol : chloroform = 1 : 10)
° Angle of rotation ; $[\alpha]_D$ = -42.0°
(C = 0.76, methanol)
° Elemental analysis ; $C_{35}H_{48}N_4O_6 \cdot 0.5H_2O$

| | C | H | N |
|---|---|---|---|
| calculated (%) | 66.75 | 7.84 | 8.90 |
| found (%) | 66.99 | 7.65 | 8.78 |

3) Synthesis of Z-Pro-Trp-Tle-Leu-OBu$^t$

418 mg of Z-Pro-OH was dissolved in 10 ml of THF, followed by cooling to -20° C. 0.184 ml of N-methylmorpholine and 0.160 ml of ethyl chlorocarbonate were added thereto. After 5 minutes, a solution of 770 mg of H-Trp-Tle-Leu-OBu$^t$ prepared by the catalytic reduction of Z-Trp-Tle-Leu-OBu$^t$ in the presence of Pd-C in 5 ml of THF was added thereto. The obtained mixture was stirred at -5° C for 2 hours and filtered to remove precipitates. The filtrate was concentrated and the residue was dissolved in ethyl acetate to obtain a solution. This solution was washed with an aqueous solution of $NaHCO_3$ and water successively and evaporated to dryness to obtain 1.1 g of Z-Pro-Trp-Tle-Leu-OBu$^t$ as a powder.

° TLC ; Rf value = 0.69
(methanol : chloroform = 1 : 6)
° Angle of rotation; $[\alpha]_D$ = -88.7°
(C = 0.63, dichloromethane)
° Elemental analysis ; $C_{40}H_{55}N_5O_7 \cdot 0.3H_2O$

| | C | H | N |
|---|---|---|---|
| calculated (%) | 66.42 | 7.75 | 9.68 |
| found (%) | 66.36 | 7.61 | 9.65 |

4) Synthesis of Z-Arg(Tos)-Pro-Trp-Tle-Leu-OBu$^t$

3.6 g of H-Pro-Trp-Tle-Leu-OBu$^t$ prepared by the catalytic reduction of Z-Pro-Trp-Tle-Leu-OBu$^t$ in the presence of Pd-C and 2.85 g of Z-Arg(Tos)-OH were dissolved in 50 ml of THF, followed by cooling to 5° C. 1.25 g of N-hydroxybenzotriazole and 1.4 g of dicyclohexylcarbodiimide were added thereto successively. The obtained mixture was stirred at 5° C overnight and filtered to remove precipitates. The obtained filtrate was concentrated and the residue was dissolved in ethyl acetate to obtain a solution. This solution was washed with an aqueous solution of $Na_2CO_3$ and water and evaporated to dryness. The obtained residue was purified by silica gel column chromatography to obtain 3.46 g of Z-Arg(Tos)-Pro-Trp-Tle-Leu-OBu$^t$.

- TLC ; Rf value = 0.25
(methanol : chloroform = 1 : 8)
- Angle of rotation; $[\alpha]_D$ = -41.9°
(C = 0.67, dichloromethane)
- Elemental analysis ; $C_{53}H_{73}N_9O_{10}S \cdot 1.5H_2O$

| | C | H | N |
|---|---|---|---|
| calculated (%) | 60.32 | 7.26 | 11.95 |
| found (%) | 60.57 | 7.12 | 12.08 |

5) Synthesis of Boc-D-Lys(Cl-Z)-Arg(Tos)-Pro-Trp-Tle-Leu-OBu$^t$

1.63 g of Boc-D-Lys(Cl-Z)-OH-TBA was dissolved in water and ethyl acetate in the presence of 3.4 ml of N-$H_2SO_4$ and the ethyl acetate layer was washed with water and evaporated to dryness to obtain an oil. This oil was dissolved in 30 ml of THF to obtain a solution. This solution was cooled to -20° C, followed by the addition thereto of 0.368 ml of N-methylmorpholine and 0.319 ml of ethyl chlorocarbonate. After 5 minutes, a solution of 2.99 g of H-Arg(Tos)-Pro-Trp-Tle-Leu-OBu$^t$ prepared by the catalytic reduction of Z-Arg(Tos)-Pro-Trp-Tle-Leu-OBu$^t$ in the presence of Pd-C in 10 ml of THF was added thereto. The obtained mixture was stirred at -5° C for 2 hours and filtered to remove precipitates. The filtrate was evaporated to dryness to obtain 3.7 g of Boc-D-Lys(Cl-Z)-Arg(Tos)-Pro-Trp-Tle-Leu-OBu$^t$.

- TLC ; Rf value = 0.61
(methanol : chloroform = 1 : 8)
- Angle of rotation ; $[\alpha]_D$ = 51.4°
(C = 0.18, dichloromethane)
- Elemental analysis ; $C_{64}H_{92}N_{11}O_{13}SCl \cdot 1H_2O$

| | C | H | N |
|---|---|---|---|
| calculated (%) | 58.72 | 7.24 | 11.77 |
| found (%) | 58.48 | 7.09 | 11.52 |

6) Synthesis of H-D-Lys-Arg-Pro-Trp-Tle-Leu-OH

3.0 g of Boc-D-Lys(Cl-Z)-Arg(Tos)-Pro-Trp-Tle-Leu-OBu$^t$ was dissolved in 100 ml of HF in a hermetically, closed HF reactor in the presence of 1.5 ml of anisole at -10° C, followed by stirring for one hour. The HF was distilled off. The obtained residue was washed with ether and dissolved in water to obtain a

solution. This solution was treated with Amberlite IRA-93 acetic acid form) and freeze-dried to obtain a crude peptide. The crude peptide was purified with an ion-exchanger (CM-Toyopearl, 2.5 ∅ x 24.5 cm, $H_2O$-0.4 M ammonium acetate, the gradient method) to obtain 760 mg of H-D-Lys-Arg-Pro-Trp-Tle-Leu-OH as its acetic acid salt.

- TLC ; Rf value = 0.43

(butanol : acetic acid : water = 4 : 1 : 1)

- Angle of rotation ; $[\alpha]_D$ = -79.2°

(C = 1, 1% acetic acid)

- Mass spectrometric analysis (FAB) ; 840 $[(M+H)^+]$
- Amino acid analysis ; Pro 1.05(1), Leu 0.95(1), Lys 1.02(1), Trp 0.93(1), Arg 1.05(1)

Example 6

Synthesis of H(Me)Arg-Lys-Pro-Trp-Tle-Leu-OEt

1) Synthesis of Z-Pro-Trp-Tle-Leu-OEt

1.11 g of Z-Pro-OH was dissolved in 15 ml of THF to obtain a solution. This solution was cooled to -20°C, followed by the addition thereto of 0.49 ml of N-methylmorpholine and 0.43 ml of ethyl chlorocarbonate. After 5 minutes, a solution of 0.21 g of H-Trp-Tle-Leu-OEt prepared by the catalytic reduction of Z-Trp-Tle-Leu-OEt in the presence of Pd-C in 10 ml of THF was added thereto. The obtained mixture was stirred at -5°C for 2 hours and filtered to remove precipitates. The filtrate was concentrated and the obtained residue was dissolved in ethyl acetate to obtain a solution. This solution was washed with an aqueous solution of $Na_2CO_3$ and water and evaporated to dryness. The obtained residue was purified by silica gel column chromatography to obtain 30.9 g of Z-Pro-Trp-Tle-Leu-OEt as an amorphous substance.

- TLC ; Rf value = 0.36

(methanol : chloroform = 1 : 20)

- Angle of rotation ; $[\alpha]_D$ = 41.1° (C = 1, DMF)
- Elemental analysis ; $C_{38}H_{51}N_5O_7$

| | C | H | N |
|---|---|---|---|
| calculated (%) | 66.16 | 7.45 | 10.15 |
| found (%) | 65.89 | 7.46 | 10.05 |

2) Synthesis of Z-Lys(Boc)-Pro-Trp-Tle-Leu-OEt

1.66 g of Z-Lys(Boc)-OH was dissolved in 30 ml of THF, followed by cooling to -20°C. 0.48 ml of N-methylmorpholine and 0.42 ml of ethyl chlorocarbonate were added. After 5 minutes, a solution of 2.42 g of H-Pro-Trp-Tle-Leu-OEt prepared by the catalytic reduction of Z-Pro-Trp-Tle-Leu-OEt in the presence of Pd-C in 20 ml of THF was added. The obtained mixture was stirred at -5°C for 2 hours and filtered to remove precipitate. The residue was dissolved in ethyl acetate to obtain a solution. This solution was washed with an aqueous solution of $Na_2CO_3$ and water successively and evaporated to dryness. The obtained residue was purified by silica gel column chromatography to obtain 3.03 g of Z-Lys(Boc)-Pro-Trp-Tle-Leu-OEt as an amorphous substance.

- TLC ; Rf value = 0.50

(methanol : dichloromethane = 1 : 10)

- Angle of rotation ; $[\alpha]_D$ = -39.3° (C = 1, DMF)
- Elemental analysis ; $C_{49}H_{72}N_7O_{10} \cdot 0.3H_2O$

| | C | H | N |
|---|---|---|---|
| calculated (%) | 63.66 | 7.91 | 10.60 |
| found (%) | 63.55 | 7.83 | 10.55 |

3) Synthesis of Boc-(Me)Arg(Mtr)-Lys(Boc)-Pro-Trp- Tle-Leu-OEt

1.79 g of Boc-(Me)Arg(Mtr)-OH prepared from H-Arg(Mtr)-OH according to the method of P. Quitt et al. [Helvetica Chemica Acta, 32, 327 (1963)] was dissolved in 300 ml of THF to obtain a solution. This solution was cooled to -20°C, followed by the addition thereto of 0.39 ml of N-methylmorpholine and 0.34 ml of ethyl chlorocarbonate. After 5 minutes, a solution of 2.54 g of H-Lys(Boc)-Pro-Trp-Tle-Leu-OEt prepared by the catalytic reduction of Z-Lys(Boc)-Pro-Trp-Tle-Leu-OEt in the presence of Pd-C in 20 ml of THF was added thereto. The obtained mixture was stirred at -5°C for 2 hours and filtered to remove precipitates. The filtrate was concentrated and the residue was dissolved in ethyl acetate to obtain a solution. This solution was washed with an aqueous solution of $Na_2CO_3$ and water and evaporated to dryness. The obtained residue was purified by silica gel column chromatography to obtain 2.33 g of Boc-(Me)Arg(Mtr)-Lys(Boc)-Pro-Trp-Tle-Leu-OEt.

- TLC ; Rf value = 0.34
- Angle of rotation ; $[\alpha]_D$ = -33.6° (C = 1, DMF)
- Elemental analysis ; $C_{63}H_{102}N_{11}O_{14}S \cdot 0.3H_2O$

| | C | H | N |
|---|---|---|---|
| calculated (%) | 59.35 | 8.11 | 12.08 |
| found (%) | 59.18 | 7.96 | 11.96 |

4) Synthesis of H(Me)Arg-Lys-Pro-Trp-Tle-Leu-OEt

1.2 g of Boc-(Me)Arg(Mtr)-Lys(Boc)-Pro-Trp-Tle-Leu-OEt and 1.2 ml of anisole were dissolved in 24 ml of $CF_3COOH$ to obtain a solution. The solution was stirred at 30°C for 4 hours and distilled to remove the $CF_3COOH$. The obtained residue was washed with ether and filtered to obtain a crude peptide. This crude peptide was purified by high-performance liquid chromatography [Nucleosil-5C18, 2.0 ⌀ x 25 cm, eluted with 0.015% HCl ($H_2O$-$CH_3CN$ = 84 : 16)] and freeze-dried to obtain 350 mg of H(Me)Arg-Lys-Pro-Trp-Tle-Leu-OEt as its hydrochloride in the form of powder.

- TLC ; Rf value = 0.71
(butanol : acetic acid : water = 2 : 1 : 1)
- Angle of rotation ; $[\alpha]_D$ = -71.3°
(C = 0.8, 1% acetic acid)
- Mass spectrometric analysis (FAB) ; 854 [$(M+H)^+$]
- Amino acid analysis ; Pro 1.07(1), Leu 0.9(1), Trp 0.96(1)

Example 7

Synthesis of H(Me)Arg-Lys-Pro-Trp-Tle-Leu-OH

2.97 g of H(Me)Arg-Lys-Pro-Trp-Tle-Leu-OEt was dissolved in a mixture comprising 20 ml of $H_2O$ and ml of MeOH, followed by the addition thereto of 9.25 ml of 2N-NaOH. The obtained mixture was stirred for 2 hours, neutralized with N-HCl and concentrated. The residue was purified by high-performance liquid chromatography [Chemcosorb 5-ODS-H, 2.0 ⌀ x 25 cm, eluted with 0.015% HCl ($H_2O$-$CH_3CN$ = 90 : 10)] to obtain 2.4 g of H(Me)Arg-Lys-Pro-Trp-Tle-Leu-OH as its hydrochloride in the form of powder.

- TLC ; Rf value = 0.39

(butanol : acetic acid : water = 4 : 1 : 1)

- Angle of rotation ; $[\alpha]_D$ = -74.5°

(C = 0.9, 1% acetic acid)

- Mass spectrometric analysis (FAB) ; 826 $[(M+H)^+]$
- Amino acid analysis ; Pro 1.13(1), Leu 0.96(1), Trp 0.91(1)

Examples 8 to 161

The compounds shown in Table 2 were each prepared by an ordinary liquid-phase method in a similar manner to the one described in Examples 1 to 7. More precisely, the compounds were each prepared by synthesizing a peptide by a process which comprised stepwise condensation of amino acids by the use of an ester of C-terminal amino acid as a starting material according to the mixed anhydride method or the DCC-HOBt or DCC-HOSu method, or a process which comprised stepwise synthesis of a derivative comprising the 8-th to 10-th residues (Arg-Arg-Pro) of neurotensin and a derivative comprising the 11-th to 13-th residues (Tyr-Ile-Leu) thereof each from its C-terminal and binding both the derivatives by fragment condensation such as the mixed anhydride method or the DCC-HOBt or DCC-HOSu method, eliminating all the protective groups of the peptide by treatment with hydrogen fluoride (HF) or $CF_3COOH$ and purifying the resulting peptide by ion-exchange chromatography or preparative high-performance liquid chromatography.

The compounds listed in Table 2 were obtained as its acid addition salt such as acetic acid salt and hydrochloride. Addition of the salts depends on a purification method when it is needed. In Table 2, each compound is shown in its salt-free form.

The angle of rotation $[\alpha]_D$, Rf value of TLC and results of amino acid analysis with respect to the obtained peptides are shown in Table 3.

Table 2

| Example No. | Compound |
|---|---|
| 8 | Arg-Arg-D-Pro-Tyr-Ile-Leu-OH |
| 9 | Arg-Lys-Pro-Tyr-Ile-Leu-OH |
| 10 | H-D-Arg-Arg-Pro-Tyr-Ile-D-Leu-OH |
| 11 | H-D-Arg-Arg-Pro-Phe-Ile-Leu-OH |
| 12 | H-D-Arg-Arg-Pro-Tyr-Leu-Leu-OH |
| 13 | Gb-Arg-Pro-Tyr-Ile-Leu-OEt |
| 14 | H-D-Arg-Arg-Pro-Trp-Ile-Leu-OEt |
| 15 | Gb-Arg-Pro-Tyr-Ile-(Me)Leu-OH |
| 16 | Gb-Arg-Pro-Pgl(p-OH)-Ile-Leu-OEt |
| 17 | Gb-Arg-Pro-Pgl-Ile-Leu-OEt |
| 18 | Ah-Arg-Pro-Trp-(Me)Leu-Leu-OEt |
| 19 | Gb-Arg-Pro-Trp-Nle-Leu-OEt |
| 20 | Gb-Arg-Pro-Trp-Ile-Leu-OEt |
| 21 | H-(Tos)Arg-Arg-Pro-Tyr-Ile-Leu-OH |
| 22 | H-D-Arg-Arg-Pro-Tyr-Ile-Leu-$NH_2$ |
| 23 | Ah-Arg-Pro-Trp-(Me)Phe-Leu-OEt |
| 24 | Ah-Arg-Pro-Trp-Pgl-Phe-OEt |
| 25 | Ah-Arg-Pro-Trp-Ser-Leu-OEt |

Table 2 (cont'd)

| Example No. | Compound |
|---|---|
| 26 | Gb-homoArg-Pro-Trp-Pgl-Leu-OEt |
| 27 | Gb-Arg-D-Pro-Trp-Pgl-Leu-OEt |
| 28 | Ah-Arg-Pro-Trp-Nva-Leu-OEt |
| 29 | Ah-Arg-Pro(S)-Trp-Pgl-Leu-OEt |
| 30 | Ah-D-Arg-Pro-Trp-Pgl-Leu-OEt |
| 31 | Ah-Arg-Pro(OH)-Trp-Pgl-Leu-OEt |
| 32 | GABA-Arg-Pro-Trp-Pgl-Leu-OEt |
| 33 | Ao-Arg-Pro-Trp-Pgl-Leu-OEt |
| 34 | Ah-Arg-Pro-Trp-Pgl-Leu-OMe |
| 35 | Ah-Arg-Pro-Trp-Pgl-Leu-NHEt |
| 36 | Ah-Arg-Pro-Trp-cLeu-Leu-OEt |
| 37 | Gh-Arg-Pro-Trp-Pgl-Leu-OMe |
| 38 | Ah-Orn-Pro-Trp-Pgl-Leu-OEt |
| 39 | Gp-Arg-Pro-Trp-Pgl-Leu-OEt |
| 40 | H-D-Arg-Arg-Pro-Trp-Pgl-Leu-OMe |
| 41 | Ah-Arg-Pro-Trp-Tle-Leu-OMe |
| 42 | H-(Me)Arg-Arg-Pro-Trp-Pgl-Leu-OEt |
| 43 | Ah-Arg-Pro-Tyr-Pgl-Leu-OEt |

Table 2 (cont'd)

| Example No. | Compound |
|---|---|
| 44 | H-D-Lys-Arg-Pro-Trp-Pgl-Leu-OEt |
| 45 | Ah-Arg-Pro-Trp-Pgl-Leu-$NH_2$ |
| 46 | H-D-Lys-Arg-Pro-Trp-Tle-Leu-OMe |
| 47 | H-D-Lys-Arg-Pro-Trp-Tle-Leu-$NH_2$ |
| 48 | Ac-D-Arg-Arg-Pro-Tyr-Ile-Leu-OEt |
| 49 | H-D-Lys-Arg-Pro-Trp-Tle-Leu-$N(Me)_2$ |
| 50 | H-D-Lys-Arg-Pro-Trp-Tle-Leu-OEt |
| 51 | H-D-Lys-Lys-Pro-Trp-Tle-Leu-OMe |
| 52 | H-(Me)Arg-Arg-Pro-Trp-Tle-Leu-OMe |
| 53 | H-D-Lys-Arg-Pro-Trp-Tle-Leu-OnHe |
| 54 | H-D-Lys-Arg-Pro-Trp-Leu(γ-Me)-Leu-OEt |
| 55 | H-D-Lys-Arg-Pro-Trp-Aib-Leu-OEt |
| 56 | H-D-Lys-Arg-Pro-Tyr-Tle-Leu-OEt |
| 57 | H-D-Lys-Arg-Pro-Nal-Tle-Leu-OEt |
| 58 | H-D-Lys-Arg-Pro-Trp-Leu(α-Me)-Leu-OEt |
| 59 | H-D-Lys-Arg-Pro-L-(Me)Trp-Tle-Leu-OEt |
| 60 | H-D-Lys-Arg-Pro-Bal-Tle-Leu-OH |
| 61 | H-D-Lys-Arg-Pro-$\Delta^2$Tyr-Tle-Leu-OH |

Table 2 (cont'd)

| Example No. | Compound |
|---|---|
| 62 | H-D-Arg-Arg-Pro-Tyr-Tle-Leu-OH |
| 63 | H-D-Lys-Arg-Pro-Tyr-Tle-Leu-OH |
| 64 | H-(Me)Arg-Arg-Pro-Trp-(Me)Ile-Leu-OH |
| 65 | H-D-Lys-Arg-Pro-Trp-(Me)Ile-Leu-OH |
| 66 | Ah-Arg-Pro-Trp-(Me)Ile-Leu-OH |
| 67 | H-D-Lys-Arg-Pro-Tyr(-CO 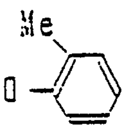 )-Ile-Leu-OH |
| 68 | H-(Me)Arg-Arg-Pro-Trp-Tle-Leu-OH |
| 69 | H-D-Lys-Arg-Pro-Trp-(Me)Ile-Tle-OH |
| 70 | H-D-Lys-Arg-Pro-Tyr(-CO 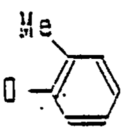 )-Tle-Leu-OH |
| 71 | H-D-Lys-Arg-Pro-Tyr(-CO 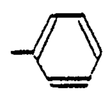 )-Tle-Leu-OH |
| 72 | H-(Me)Arg-Lys-Pro-Tyr-Tle-Leu-OH |
| 73 | H-(Me)Arg-Lys-Pro-Tyr(-CO 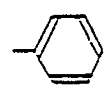 )-Tle-Leu-OH |

Table 2 (cont'd)

| Example No. | Compound |
|---|---|
| 74 | H-(Me)Lys-Arg-Pro-Tyr-Tle-Leu-OH |
| 75 | H-(Me)Lys-Arg-Pro-Tyr(-CO 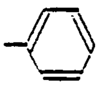)-Tle-Leu-OH |
| 76 | H-(Me)Lys-Arg-Pro-Tyr(-CO 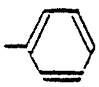)-Tle-Leu-OEt |
| 77 | H-(Me)Lys-Arg-Pro-Trp-Tle-Leu-OH |
| 78 | H-(Me)Arg-Lys-Pro-Tyr(-CO 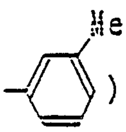)-Tle-Leu-OH |
| 79 | H-(Me)Arg-Lys-Pro-Tyr(-CO-CH($CH_3$)$_2$)-Tle-Leu-OH |
| 80 | H-(Me)Arg-Lys-Pro-Tyr(-CO 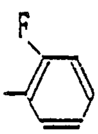)-Tle-Leu-OH |
| 81 | H-(Me)Arg-Lys-Pro-Tyr(-CO 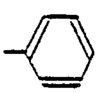)-Tle-Leu-OH |
| 82 | H-(Me)Arg-Lys-Pro-Tyr(-CO-$CH_2$ 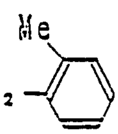)-Tle-Leu-OH |

Table 2 (cont'd)

| Example No. | Compound |
|---|---|
| 83 | H-(Me)Arg-Lys-Pro-Tyr(-CO-$CH_2$-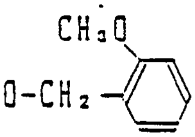)-Tle-Leu-OH |
| 84 | H-D-Lys-Arg-Pro-Tyr-Ile-Leu-OH |
| 85 | Gb-Arg-Pro-Tyr-Ile-Leu-OH |
| 86 | H-(Me)Arg-Lys-Pro-Trp-Tle-Leu-OcHe |
| 87 | H-(Me)Arg-Lys-Pro-Trp-Tle-Leu-OisoPr |
| 88 | H-(Me)Arg-Lys-Pro-Tyr(-CO-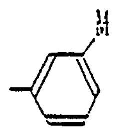)-Tle-Leu-OEt |
| 89 | H-(Me)Arg-Lys-Pro-Tyr(-CO-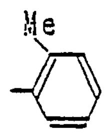)-Tle-Leu-OEt |
| 90 | H-(Me)Arg-Lys-Pro-Tyr(-CO-$CH(CH_3)_2$)-Tle-Leu-OEt |
| 91 | H-(Me)Arg-Lys-Pro-Trp-Tle-Ile-OEt |
| 92 | H-(Me)Arg-Lys-Pro-Trp-Tle-Leu-$O(CH_2)_{12}CH_3$ |
| 93 | H-D-Arg-Lys-Pro-Trp-Tle-Leu-OEt |
| 94 | H-(Me)Arg-Lys-Pro-Trp-Tle-Phe-OEt |
| 95 | H-(Me)Arg-Lys-Pro-Trp-Tle-Val-OEt |

Table 2 (cont'd)

| Example No. | Compound |
|---|---|
| 96 | H-(Me)Arg-Lys-Pro-Trp-Tle-Leu-OMe |
| 97 | H-(Me)Arg-Lys-Pro-Trp-Tle-Leu-OisoBu |
| 98 | H-(Me)Arg-Lys-Pro-Trp-Tle-Leu-OnPr |
| 99 | H-(Me)Arg-Lys-Pro-Trp-Tle-Leu-O$(CH_2)_{17}CH_3$ |
| 100 | H-(Me)Arg-Lys-Pro-Tyr(-Et)-Tle-Leu-OEt |
| 101 | H-(Me)Lys-Arg-Pro-Tyr(-Et)-Tle-Leu-OEt |
| 102 | H-(Me)Arg-Lys-Pro-Trp-Tle-Tle-OEt |
| 103 | H-(Me)Arg-Lys-Pro-Trp-Tle-Leu-O$(CH_2)_2$OH |
| 104 | Ac-D-Arg-Lys-Pro-Trp-Tle-Leu-OEt |
| 105 | H-D-Arg-Arg-Pro-Tyr-Ile-Leu-OEt |
| 106 | Ac(Me)Arg-Lys-Pro-Trp-Tle-Leu-OEt |
| 107 | H-(Me)Arg-Lys-Pro-Trp-Pro-Leu-OEt |
| 108 | H-(Me)Arg-Lys-Pro-Trp-Tle-D-Leu-OEt |
| 109 | H-(Me)Arg-Lys-Pro-Trp-Tle-Aib-OEt |
| 110 | H-(Me)Arg-Lys-Pro-Trp-Tle-Leu-O$(CH_2)_2$OAc |
| 111 | H-D-Lys-Arg-Pro-Trp-Tle-Leu-O$(CH_2)_2$OH |
| 112 | $CH_3$OCOCO-(Me)Arg-Lys-Pro-Trp-Tle-Leu-OEt |

Table 2 (cont'd)

| Example No. | Compound |
|---|---|
| 113 | H-Inp-Arg-Pro-Trp-Tle-Leu-OEt |
| 114 | Bz-(Me)Arg-Lys-Pro-Trp-Tle-Leu-OEt |
| 115 | H-D-Orn-Arg-Pro-Trp-Tle-Leu-OEt |
| 116 | H-(Me)Arg-Orn-Pro-Trp-Tle-Leu-OEt |
| 117 | H-D-Lys-homoArg-Pro-Trp-Tle-Leu-OEt |
| 118 | H-(Me)Arg-Lys-Pro-Trp-(Me)Tle-Leu-OEt |
| 119 | H-Paa-Arg-Pro-Trp-Tle-Leu-OEt |
| 120 | Ac-(Me)Arg-Lys-Pro-Trp-Tle-Leu-OH |
| 121 | Bz-(Me)Arg-Lys-Pro-Trp-Tle-Leu-OH |
| 122 | H-(Me)Arg-Lys-Pro-Trp-Tle-D-Leu-OH |
| 123 | H-D-Orn-Arg-Pro-Trp-Tle-Leu-OH |
| 124 | H-(Me)Arg-Orn-Pro-Trp-Tle-Leu-OH |
| 125 | H-(Me)Arg-Lys-Pro-Trp-Tle-Tle-OH |
| 126 | Ac-D-Arg-Arg-Pro-Tyr-Ile-Leu-$NH_2$ |
| 127 | Ac-D-Arg-Lys-Pro-Trp-Tle-Leu-OH |
| 128 | Ah-Arg-Pro-Trp-Pgl-Leu-OH |
| 129 | H-D-Lys-Arg-Pro-Nal-Tle-Leu-OH |

Table 2 (cont'd)

| Example No. | Compound |
|---|---|
| 130 | H-D-Lys-homoArg-Pro-Trp-Tle-Leu-OH |
| 131 | H-Inp-Arg-Pro-Trp-Tle-Leu-OH |
| 132 | H-(Me)Arg-Lys-$\Delta^3$Pro-Trp-Tle-Leu-OH |
| 133 | H-(Me)Arg-Lys-$\Delta^3$Pro-Trp-Tle-Leu-OEt |
| 134 | H-D-Arg-Lys-Pro-Trp-Tle-Leu-OH |
| 135 | 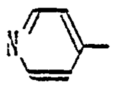 CO-D-Arg-Lys-Pro-Trp-Tle-Leu-OEt |
| 136 | 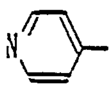 CO-(Me)Arg-Lys-Pro-Trp-Tle-Leu-OEt |
| 137 | HO-$CH_2$-CO-D-Arg-Lys-Pro-Trp-Tle-Leu-OEt |
| 138 | 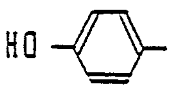 CO-D-Arg-Lys-Pro-Trp-Tle-Leu-OEt |
| 139 | 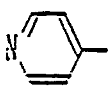 CO-D-Arg-Lys-Pro-Trp-Tle-Leu-OH |
| 140 | 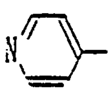 CO-(Me)Arg-Lys-Pro-Trp-Tle-Leu-OH |
| 141 | HO-$CH_2$-CO-D-Arg-Lys-Pro-Trp-Tle-Leu-OH |
| 142 | 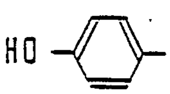 CO-D-Arg-Lys-Pro-Trp-Tle-Leu-OH |

Table 2 (cont'd)

| Example No. | Compound |
|---|---|
| 143 | 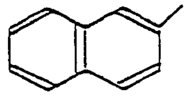 CO-D-Arg-Lys-Pro-Trp-Tle-Leu-OH |
| 144 | H-D-Lys-Lys-Pro-Trp-Tle-Leu-OH |
| 145 | 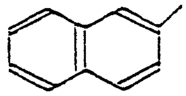 CO-D-Arg-Lys-Pro-Trp-Tle-Leu-OEt |
| 146 | 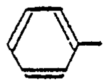 CO-D-Orn-Arg-Pro-Trp-Tle-Leu-OEt |
| 147 | 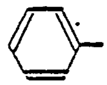 CO-D-Orn-Arg-Pro-Trp-Tle-Leu-OH |

Table 2 (contineued)

| example No. | compound |
|---|---|
| 148 | H-D-Lys-Orn-Pro-Trp-Tle-Leu-OEt |
| 149 | H-Inp-Lys-Pro-Trp-Tle-Leu-OEt |
| 150 | H-D-Orn-Lys-Pro-Trp-Tle-Leu-OEt |
| 151 | H-D-Inp-Orn-Pro-Trp-Tle-Leu-OEt |
| 152 | H-D-Orn-Orn-Pro-Trp-Tle-Leu-OEt |
| 153 | H-Paa-Lys-Pro-Trp-Tle-Leu-OEt |
| 154 | H-Paa-Orn-Pro-Trp-Tle-Leu-OEt |
| 155 | H-D-Lys-Orn-Pro-Trp-Tle-Leu-OH |
| 156 | H-Inp-Lys-Pro-Trp-Tle-Leu-OH |
| 157 | H-D-Orn-Lys-Pro-Trp-Tle-Leu-OH |
| 158 | H-Inp-Orn-Pro-Trp-Tle-Leu-OH |
| 159 | H-D-Orn-Orn-Pro-Trp-Tle-Leu-OH |
| 160 | H-Paa-Lys-Pro-Trp-Tle-Leu-OH |
| 161 | H-Paa-Orn-Pro-Trp-Tle-Leu-OH |

Table 3

| Example No. | [α]D *1 | Amino acid analysis *2 | | | | | | TLC Rf value *3 |
|---|---|---|---|---|---|---|---|---|
| 8 | −3.3 | Pro 1.07 | Ile 0.85 | Leu 0.95 | Tyr 0.98 | Arg 2.15 | | (C)0.41 |
| 9 | −52.2 | Pro 0.91 | Ile 0.97 | Leu 1.03 | Tyr 0.98 | Lys 1.03 | Arg 1.07 | (A)0.27, (C)0.29 |
| 10 | −47.4 | Pro 0.97 | Ile 0.88 | Leu 0.99 | Tyr 0.99 | Arg 2.18 | | (C)0.39 |
| 11 | −88.1 | Pro 0.98 | Ile 0.88 | Leu 0.98 | Phe 0.98 | Arg 2.16 | | (A)0.33, (B)0.67 |
| 12 | −85.2 | Pro 0.99 | Leu 2.08 | Tyr 1.05 | Arg 2.20 | | | (A)0.33, (B)0.66 |
| 13 | −77.0 | Pro 1.01 | Ile 0.93 | Leu 1.01 | Tyr 0.98 | Arg 1.08 | | (C)0.55 |
| 14 | −76.4 | Pro 0.96 | Ile 0.90 | Leu 1.00 | Trp 0.91 | Arg 2.24 | | (A)0.36, (B)0.70 |
| 15 | −83.3 | Pro 1.00 | Ile 0.95 | Leu 0.95 | Arg 1.09 | | | (C)0.53 |
| 16 | −43.6 | Pro 1.00 | Ile 0.96 | Leu 0.99 | Arg 1.05 | | | (B)0.73 |
| 17 | −54.2 | Pro 0.99 | Ile 0.85 | Leu 1.00 | Arg 1.16 | | | (C)0.55 |
| 18 | −75.5 | Pro 1.01 | Leu 1.03 | Trp 1.08 | Arg 1.11 | | | (A)0.39 |
| 19 | −72.6 | Pro 1.01 | Leu 0.98 | Trp 0.89 | Arg 1.11 | | | (C)0.59 |
| 20 | −80.8 | Pro 1.03 | Ile 0.94 | Leu 1.02 | Trp 0.87 | Arg 1.14 | | (C)0.51 |
| 21 | −57.4 | Pro 0.98 | Ile 0.96 | Leu 1.05 | Tyr 1.00 | | | |
| 22 | −71.2 | Pro 0.99 | Ile 0.90 | Leu 0.99 | Tyr 0.97 | Arg 2.16 | | (C)0.40 |
| 23 | −82.5 | Pro 1.00 | Leu 1.00 | Trp 1.00 | Arg 1.00 | | | (C)0.57 |
| 24 | −27.2 | Pro 1.07 | Phe 0.97 | Trp 0.89 | Arg 1.07 | | | (C)0.55 |
| 25 | −68.7 | Ser 0.92 | Pro 1.00 | Leu 1.06 | Trp 0.98 | Arg 1.09 | | (A)0.32, (B)0.66 |
| 26 | −46.4 | Pro 1.05 | Leu 1.04 | Trp 0.91 | | | | (C)0.57 |
| 27 | +35.8 | Pro 1.04 | Leu 1.08 | Trp 0.85 | Arg 1.03 | | | (C)0.54 |
| 28 | −77.4 | Pro 1.01 | Leu 1.02 | Trp 0.83 | Arg 1.14 | | | (C)0.54 |

Table 3 (cont'd)

| Example No. | (α)D *1 | Amino acid analysis *2 | | | | | | TLC Rf value *3 |
|---|---|---|---|---|---|---|---|---|
| 29 | −57.8 | Leu 1.07 | Trp 0.92 | Arg 1.01 | | | | (A)0.41, (C)0.65 |
| 30 | −24.8 | Pro 0.98 | Leu 1.08 | Trp 0.94 | Arg 1.00 | | | (C)0.49 |
| 31 | −37.6 | Leu 1.07 | Trp 0.87 | Arg 1.06 | | | | (A)0.24, (C)0.60 |
| 32 | −66.9 | Pro 1.05 | Leu 1.10 | Trp 0.80 | Arg 1.06 | | | (C)0.25 |
| 33 | −46.5 | Pro 1.03 | Leu 1.08 | Trp 0.86 | Arg 1.02 | | | (C)0.47 |
| 34 | −41.5 | Pro 0.99 | Leu 1.06 | Trp 0.84 | Arg 1.11 | | | (C)0.46 |
| 35 | −44.2 | Pro 0.98 | Leu 1.05 | Trp 0.86 | Arg 1.11 | | | (A)0.24 |
| 36 | −66.7 | Pro 0.94 | Leu 1.06 | Trp 0.94 | Arg 1.06 | | | (A)0.38, (C)0.57 |
| 37 | −83.5 | Pro 1.13 | Leu 0.96 | Trp 0.87 | Arg 1.05 | | | (C)0.44 |
| 38 | −71.5 | Pro 1.03 | Leu 1.15 | Trp 0.83 | | | | (C)0.69 |
| 39 | −44.0 | Pro 1.08 | Leu 1.02 | Trp 0.84 | Arg 1.04 | | | (C)0.51 |
| 40 | −45.2 | Pro 0.92 | Leu 1.07 | Trp 0.88 | Arg 1.05 | | | (A)0.31 |
| 41 | −82.0 | Pro 0.98 | Leu 0.95 | Trp 0.90 | Arg 1.18 | | | (C)0.47 |
| 42 | −63.8 | Pro 1.02 | Leu 1.01 | Trp 0.97 | | | | (A)0.29, (B)0.73 |
| 43 | −44.1 | Pro 1.01 | Leu 1.02 | Tyr 0.96 | Arg 1.01 | | | (A)0.36, (B)0.65 |
| 44 | −54.9 | Pro 0.92 | Leu 1.04 | Lys 1.02 | Trp 0.95 | Arg 1.08 | | (A)0.29, (B)0.64 |
| 45 | −54.5 | Pro 0.95 | Leu 1.08 | Trp 0.86 | Arg 1.11 | | | (A)0.32 |
| 46 | −77.4 | Pro 1.05 | Leu 0.91 | Lys 0.96 | Arg 1.08 | | | (C)0.42 |
| 47 | −80.6 | Pro 0.89 | Leu 0.97 | Lys 1.08 | Trp 0.93 | Arg 1.15 | | (A)0.26, (B)0.57 |
| 48 | −58.6 | Pro 1.00 | Ile 0.93 | Leu 1.00 | Tyr 0.98 | Arg 2.08 | | (A)0.37, (C)0.28 |
| 49 | −76.8 | Pro 1.01 | Leu 1.01 | Lys 1.03 | Trp 0.94 | Arg 1.04 | | (A)0.37, (C)0.41 |

Table 3 (cont'd)

| Example No. | $[\alpha]_D$ *1 | Amino acid analysis *2 | | | | | TLC Rf value *3 |
|---|---|---|---|---|---|---|---|
| 50 | −84.4 | Pro 0.92 | Leu 0.96 | Lys 1.05 | Trp 0.93 | Arg 1.14 | (A)0.30, (C)0.41 |
| 51 | −93.2 | Pro 1.00 | Leu 0.88 | Lys 2.24 | Trp 0.87 | | (B)0.55, (C)0.37 |
| 52 | −76.9 | Pro 1.13 | Leu 0.91 | Trp 0.97 | | | (A)0.28, (B)0.64 |
| 53 | −84.0 | Pro 1.05 | Leu 0.97 | Lys 1.03 | Trp 0.88 | Arg 1.08 | (A)0.31, (B)0.65 |
| 54 | −92.0 | Pro 0.94 | Lys 1.04 | Trp 0.94 | Arg 1.08 | | (A)0.31, (B)0.65 |
| 55 | −65.7 | Pro 0.93 | Leu 1.08 | Lys 1.03 | Trp 0.91 | Arg 1.06 | (C)0.41 |
| 56 | −77.9 | Pro 0.97 | Leu 0.90 | Tyr 0.95 | Lys 1.07 | Arg 1.10 | (C)0.42 |
| 57 | −68.9 | Pro 1.00 | Leu 0.87 | Lys 1.04 | Arg 1.09 | | (A)0.31, (B)0.63 |
| 58 | −70.5 | Pro 0.94 | Leu 1.08 | Lys 1.05 | Trp 0.84 | Arg 1.08 | (C)0.44 |
| 59 | −108.7 | Pro 0.84 | Leu 1.01 | Tyr 1.06 | Trp 1.11 | | (A)0.22, (B)0.66 |
| 60 | −84.0 | Pro 0.93 | Leu 0.91 | Lys 1.07 | Arg 1.11 | | (A)0.28, (B)0.61 |
| 61 | −10.1 | Pro 0.94 | Leu 0.99 | Lys 1.02 | Arg 1.05 | | (C)0.41 |
| 62 | −74.4 | Pro 0.99 | Leu 1.02 | Trp 0.94 | Arg 2.05 | | (C)0.41 |
| 63 | −80.1 | Pro 1.01 | Leu 0.89 | Tyr 0.95 | Trp 1.04 | Arg 1.10 | (B)0.54, (C)0.38 |
| 64 | −73.9 | Pro 1.03 | Leu 1.05 | Trp 0.92 | | | (A)0.28, (C)0.40 |
| 65 | −112.0 | Pro 0.92 | Leu 1.10 | Lys 1.03 | Trp 0.87 | Arg 1.08 | (A)0.23, (B)0.60 |
| 66 | −109.2 | Pro 1.02 | Leu 1.02 | Trp 0.90 | Arg 1.06 | | (A)0.30 |
| 67 | −74.3 | Pro 0.98 | Leu 0.92 | Tyr 0.95 | Lys 1.06 | Arg 1.09 | (B)0.65 |
| 68 | −66.0 | Pro 1.05 | Leu 0.95 | Trp 1.00 | | | (A)0.23, (C)0.43 |
| 69 | −104.7 | Pro 1.07 | Lys 1.04 | Trp 0.84 | Arg 1.05 | | (A)0.32, (C)0.41 |
| 70 | −62.8 | Pro 0.94 | Leu 0.95 | Tyr 0.98 | Lys 1.04 | Arg 1.09 | (A)0.21, (B)0.61 |

Table 3 (cont'd)

| Example No. | $[\alpha]_D$ *1 | Amino acid analysis *2 | | | | | | TLC Rf value *3 |
|---|---|---|---|---|---|---|---|---|
| 71 | −63.2 | Pro 0.97 | Leu 0.93 | Tyr 0.96 | Lys 1.04 | Arg 1.10 | | (A) 0.29, (B) 0.61 |
| 72 | −62.0 | Pro 1.04 | Leu 1.08 | Tyr 0.89 | | | | (C) 0.33 |
| 73 | −49.3 | Pro 0.98 | Leu 0.99 | Tyr 1.03 | | | | (C) 0.38 |
| 74 | −70.7 | Pro 1.05 | Leu 0.97 | Tyr 0.99 | | | | (A) 0.25, (B) 0.43 |
| 75 | −54.8 | Pro 0.99 | Leu 1.04 | Tyr 0.96 | | | | (B) 0.59 |
| 76 | −59.4 | Pro 0.89 | Leu 0.98 | Trp 1.03 | | | | (C) 0.41 |
| 77 | −89.6 | Pro 1.16 | Leu 0.97 | Tyr 0.87 | | | | (C) 0.39 |
| 78 | −53.3 | Pro 0.96 | Leu 1.01 | Tyr 1.03 | | | | (C) 0.41 |
| 79 | −55.9 | Pro 1.07 | Leu 0.96 | Tyr 0.97 | | | | (C) 0.41 |
| 80 | −59.4 | Pro 0.99 | Leu 1.00 | Tyr 1.01 | | | | (C) 0.44 |
| 81 | −57.2 | Pro 0.96 | Leu 1.01 | Tyr 1.03 | | | | (B) 0.72 |
| 82 | −53.2 | Pro 1.03 | Leu 1.10 | Tyr 0.87 | | | | (B) 0.57 |
| 83 | −62.5 | Pro 1.06 | Leu 0.95 | Tyr 0.99 | | | | (C) 0.42 |
| 84 | −58.2 | Pro 1.10 | Ile 0.88 | Leu 0.98 | Tyr 0.95 | Lys 1.03 | Arg 1.07 | (A) 0.28, (B) 0.56 |
| 85 | −74.3 | Pro 0.96 | Ile 0.91 | Leu 1.02 | Tyr 0.98 | Arg 1.13 | | (C) 0.53 |
| 86 | −67.8 | Pro 1.08 | Leu 0.99 | Trp 0.93 | | | | (A) 0.22, (B) 0.59 |
| 87 | −64.3 | Pro 1.08 | Leu 0.99 | Trp 0.93 | | | | (A) 0.23, (B) 0.58 |
| 88 | −56.2 | Pro 1.10 | Leu 0.91 | Tyr 0.99 | | | | (A) 0.24, (C) 0.45 |
| 89 | −52.8 | Pro 1.08 | Leu 0.93 | Tyr 0.99 | | | | (A) 0.25, (B) 0.59 |
| 90 | −58.9 | Pro 1.06 | Leu 0.93 | Tyr 1.00 | | | | (A) 0.22, (B) 0.58 |
| 91 | −71.1 | Pro 1.19 | Ile 0.78 | Trp 1.03 | | | | (A) 0.23, (B) 0.57 |

Table 3 (cont'd)

| Example No. | $[\alpha]_D$ *1 | Amino acid analysis *2 | | | | | | TLC Rf value *3 |
|---|---|---|---|---|---|---|---|---|
| 92 | −57.2 | Pro 1.09 | Leu 0.97 | Trp 0.94 | | | | (A)0.26, (C)0.50 |
| 93 | −94.0 | Pro 1.01 | Leu 0.89 | Lys 1.10 | Trp 0.85 | Arg 1.14 | | (A)0.24 |
| 94 | −60.4 | Pro 1.08 | Phe 0.88 | Trp 1.04 | | | | (A)0.28 |
| 95 | −64.2 | Pro 1.10 | Val 0.82 | Trp 1.08 | | | | (A)0.20, (B)0.55 |
| 96 | −75.6 | Pro 1.00 | Leu 0.98 | Trp 1.02 | | | | (A)0.20, (B)0.54 |
| 97 | −67.4 | Pro 1.03 | Leu 0.97 | Trp 1.00 | | | | (A)0.23 |
| 98 | −74.4 | Pro 1.09 | Leu 0.98 | Trp 0.93 | | | | (A)0.30 |
| 99 | −48.7 | Pro 1.08 | Leu 0.97 | Trp 0.95 | | | | (A)0.23, (C)0.51 |
| 100 | −66.5 | Pro 1.08 | Leu 0.92 | | | | | (A)0.24, (B)0.57 |
| 101 | −64.1 | Pro 1.10 | Trp 0.90 | | | | | (A)0.21, (B)0.57 |
| 102 | −66.7 | Pro 1.03 | Leu 0.97 | | | | | (A)0.21, (B)0.57 |
| 103 | −73.3 | Pro 1.08 | Leu 0.93 | Trp 0.99 | | | | (A)0.23, (B)0.51 |
| 104 | −77.6 | Pro 1.03 | Leu 0.90 | Lys 1.02 | Trp 0.92 | Arg 1.13 | | (A)0.30, (C)0.68 |
| 105 | −67.8 | Pro 0.96 | Ile 0.87 | Leu 0.98 | Tyr 0.98 | Arg 2.22 | | (C)0.43 |
| 106 | −91.2 | Pro 1.03 | Leu 0.96 | Trp 1.01 | | | | (A)0.26 |
| 107 | −107.1 | Pro 1.86 | Leu 1.09 | Trp 1.05 | | | | (A)0.25, (B)0.44 |
| 108 | −44.9 | Pro 1.06 | Leu 0.93 | Trp 1.01 | | | | (A)0.22, (B)0.56 |
| 109 | −69.2 | Pro 1.04 | Trp 0.96 | | | | | (A)0.30, (B)0.60 |
| 110 | −69.2 | Pro 1.01 | Leu 1.05 | Trp 0.94 | | | | (C)0.36, (B)0.65 |
| 111 | −91.8 | Pro 1.01 | Leu 0.95 | Lys 1.04 | Trp 0.90 | Arg 1.10 | | (B)0.60 |
| 112 | −97.7 | Pro 1.02 | Leu 0.96 | Trp 1.02 | | | | (A)0.27 |

Table 3 (cont'd)

| Example No. | [α]D *1 | Amino acid analysis *2 | | | | | | TLC Rf value *3 |
|---|---|---|---|---|---|---|---|---|
| 113 | −84.4 | Pro 0.98 | Leu 0.97 | Trp 0.92 | Arg 1.13 | | | (C)0.52 |
| 114 | −90.6 | Pro 0.98 | Leu 0.97 | Trp 1.06 | | | | (A)0.47, (C)0.67 |
| 115 | −98.0 | Pro 1.05 | Leu 0.95 | Trp 0.85 | Arg 1.15 | | | (A)0.28, (D)0.56 |
| 116 | −80.1 | Pro 1.06 | Leu 0.98 | Trp 0.95 | | | | (B)0.58, (D)0.59 |
| 117 | −94.4 | Pro 0.99 | Leu 1.01 | Lys 1.07 | Trp 0.93 | | | (B)0.69 |
| 118 | −111.5 | Pro 0.94 | Leu 1.06 | Trp 1.00 | | | | (C)0.36 |
| 119 | −90.5 | Pro 1.03 | Leu 0.94 | Trp 0.87 | Arg 1.16 | | | (C)0.53 |
| 120 | −85.3 | Pro 1.05 | Leu 0.91 | Trp 1.03 | | | | (A)0.22 |
| 121 | −106.0 | Pro 1.03 | Leu 0.92 | Trp 1.04 | | | | |
| 122 | −57.2 | Pro 1.06 | Leu 0.91 | Trp 1.03 | | | | (B)0.55 |
| 123 | −89.4 | Pro 1.07 | Leu 0.93 | Trp 0.85 | Arg 1.15 | | | (A)0.30, (D)0.52 |
| 124 | −87.9 | Pro 1.05 | Leu 0.94 | Trp 1.01 | | | | (B)0.63, (D)0.60 |
| 125 | −86.8 | Pro 1.01 | Trp 0.99 | | | | | (D)0.52 |
| 126 | −49.4 | Pro 1.01 | Ile 0.89 | Leu 0.99 | Tyr 0.96 | Arg 2.14 | | (C)0.43 |
| 127 | −57.3 | Pro 1.05 | Leu 0.92 | Lys 1.04 | Trp 0.87 | Arg 1.12 | | (A)0.46, (C)0.22 |
| 128 | −44.5 | Pro 0.89 | Leu 1.01 | Trp 1.04 | Arg 1.07 | | | (A)0.26, (C)0.26 |
| 129 | −26.3 | Pro 1.00 | Leu 0.88 | Lys 1.02 | Arg 1.10 | | | (A)0.20, (C)0.11 |
| 130 | −77.6 | Pro 1.17 | Leu 0.90 | Lys 1.04 | Trp 0.88 | | | (A)0.20, (C)0.10 |
| 131 | −75.3 | Pro 1.06 | Leu 0.91 | Trp 0.90 | Arg 1.13 | | | (A)0.235, (C)0.18 |
| 132 | −127.0 | Leu 0.99 | Trp 1.01 | | | | | (A)0.19 |
| 133 | −252.6 | Leu 1.04 | Trp 0.96 | | | | | (A)0.19 |

Table 3 (cont'd)

| Example No. | $[\alpha]_D$ *1 | Amino acid analysis *2 | | | | | | TLC Rf value *3 |
|---|---|---|---|---|---|---|---|---|
| 134 | −59.4 | Pro 1.04 | Leu 0.91 | Lys 1.05 | Trp 0.89 | Arg 1.10 | | (A) 0.21, (C) 0.10 |
| 135 | 24.0 | Pro 1.00 | Leu 0.92 | Lys 1.03 | Trp 0.89 | Arg 1.16 | | (A) 0.26, (C) 0.23 |
| 136 | −83.4 | Pro 1.09 | Leu 0.93 | Trp 0.98 | | | | (A) 0.26, (C) 0.19 |
| 137 | −76.9 | Pro 0.98 | Leu 0.91 | Lys 1.07 | Trp 0.91 | Arg 1.13 | | (A) 0.27 |
| 138 | −73.9 | Pro 0.97 | Leu 0.92 | Lys 1.07 | Trp 0.88 | Arg 1.15 | | |
| 139 | −67.1 | Pro 0.99 | Leu 0.92 | Lys 1.05 | Trp 0.91 | Arg 1.14 | | (A) 0.20, (C) 0.19 |
| 140 | −89.4 | Pro 1.03 | Leu 0.95 | Trp 1.02 | | | | (A) 0.24, (C) 0.15 |
| 141 | −63.4 | Pro 0.97 | Leu 0.93 | Lys 1.05 | Trp 0.95 | Arg 1.10 | | (A) 0.25, (C) 0.25 |
| 142 | −67.3 | Pro 0.90 | Leu 0.93 | Lys 1.07 | Trp 0.92 | Arg 1.18 | | (A) 0.33, (C) 0.40 |
| 143 | −73.8 | Pro 0.92 | Leu 0.91 | Lys 1.07 | Trp 0.93 | Arg 1.16 | | (A) 0.35, (C) 0.43 |
| 144 | −98.1 | Pro 0.94 | Leu 0.96 | Lys 2.19 | Trp 1.01 | | | (A) 0.14 |
| 145 | −93.8 | Pro 1.00 | Leu 0.91 | Lys 1.04 | Trp 0.91 | Arg 1.14 | | (A) 0.37, (C) 0.45 |
| 146 | −153.0 | Pro 0.95 | Leu 0.90 | Trp 0.94 | Arg 1.21 | | | (A) 0.35, (C) 0.43 |
| 147 | −121.3 | Pro 0.97 | Leu 0.93 | Trp 0.93 | Arg 1.17 | | | (A) 0.33, (C) 0.38 |

*1 Measurement of $[\alpha]_D$ ; 1% acetic acid

*2 The ratios of constituent amino acids were determined by amino acid analysis (hydrolysis with 2-mercaptoethanesulfonic acid) only with respect to Pro, Ile, Leu, Phe, Tyr, Lys, Trp and Arg. With respect to a peptide containing a basic N-methyl amino acid, an amide linkage containing a basic N-methyl amino acid is hardly hydrolyzed, so that no analyzed values of the amino acids which are present before and after it respectively are shown in Table 3.

*3 Solvent (A) n-butanol-acetic acid-water (4 : 1 : 5, supernatant)

(B) n-butanol-acetic acid-water (2 : 1 : 1)

(C) n-butanol-acetic acid-water (4 : 1 : 1)

(D) n-butanol-acetic acid-pyridine-water (15 : 5 : 5 : 8)

Table 3 (continued)

| No. | [α] D *1 | Amino acid analysis *2 | | | | TLC Rf value *3 |
|---|---|---|---|---|---|---|
| | | Pro | Leu | Lys | Trp | |
| 148 | -88.9 | 1.04 | 0.99 | 1.07 | 0.90 | (A)0.17, (D)0.68 |
| 149 | -84.4 | 0.97 | 0.99 | 1.12 | 0.93 | (A)0.23, (D)0.71 |
| 150 | -98.6 | 1.10 | 0.95 | 1.04 | 0.91 | (A)0.19, (D)0.65 |
| 151 | -88.8 | 1.07 | 0.95 | | 0.97 | (A)0.24, (D)0.69 |
| 152 | -93.7 | 1.10 | 0.96 | | 0.94 | (A)0.20, (D)0.65 |
| 153 | -81.1 | 1.09 | 0.93 | 1.05 | 0.93 | (A)0.22, (D)0.68 |
| 154 | -83.3 | 1.03 | 1.04 | | 0.93 | (A)0.22, (D)0.67 |
| 155 | -82.3 | 1.01 | 0.94 | 1.03 | 1.02 | (A)0.16, (D)0.57 |
| 156 | -80.2 | 1.08 | 0.92 | 1.06 | 0.94 | (A)0.20, (D)0.60 |
| 157 | -91.8 | 1.07 | 0.89 | 1.07 | 0.97 | (A)0.17, (D)0.55 |
| 158 | -83.2 | 1.06 | 0.94 | | 1.00 | (A)0.19, (D)0.56 |
| 159 | -88.2 | 1.08 | 0.97 | | 0.95 | (A)0.16, (D)0.54 |
| 160 | -73.9 | 1.08 | 0.89 | 1.07 | 0.95 | (A)0.18, (D)0.55 |
| 161 | -77.6 | 1.05 | 0.96 | | 0.99 | (A)0.19, (D)0.55 |

## Claims

1. A polypeptide represented by the following general formula and a pharmacologically acceptable salt thereof:

A-B-C-D-E-F-$R^1$

wherein A stands for a group

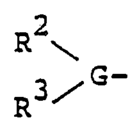

(G stands for a basic amino acid of L- or D-form, and $R^2$ and $R^3$, which may be the same or different, each stand for a hydrogen atom, a lower alkyl group or an acyl group), a group

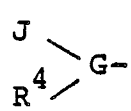

(in which G stands for a basic amino acid of L- or D-form, J stands for an amino acid of L- or D-form, and $R^4$ stands for a hydrogen atom or a lower alkyl group), an ω-aminoalkanoyl group

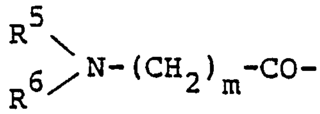

(in which $R^5$ and $R^6$, which may be the same or different, each stand for a hydrogen atom or a lower alkyl group, and m is an integer of from 1 to 10), an ω-guanidinoalkanoyl group

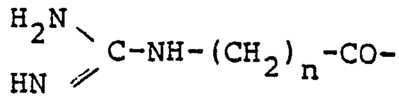

(in which n is an integer of from 1 to 10), or a group having the formula

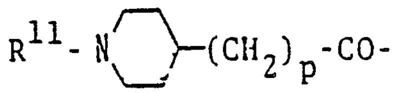

in which R11 is hydrogen or a lower alkyl, p is zero or an integer of from 1 to 10, B is a basic amino acid of L-form or an α-N-alkyl derivative thereof, C stands for L-Pro or a derivative thereof, D stands for a natural or non-natural aromatic amino acid of L-form, E stands for an amino acid of L-form or an α-N-alkyl derivative or α-C-alkyl derivative thereof, F stands for an amino acid of L- or D-form or an α-N-alkyl derivative or α-C-alkyl derivative thereof, and $R^1$ stands for a group

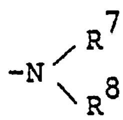

(in which $R^7$ and $R^8$, which may be the same or different, each stand for a hydrogen atom or a lower alkyl group) or a group $-O-R^9$ (in which $R^9$ stands for a hydrogen atom, an alkyl group, an alkenyl group or a group $-(CH_2)_p-O-R^{10}$ in which p is an integer of from 1 to 5 and $R^{10}$ stands for an alkyl group, an alkenyl group or an acyl group).

2. A polypeptide and a pharmacologically acceptable salt thereof according to claim 1, wherein B is an amino acid selected from the group consisting of L-Lys, L-Arg, L-homoArg and L-Orn.

3. A polypeptide and a pharmacologically acceptable salt thereof according to claim 1, wherein C is L-Pro.

4. A polypeptide and a pharmacologically acceptable salt thereof according to claim 1, wherein D is an amino acid selected from the group consisting of L-Tyr, L-Trp, L-Phe, L-Pgl, L-Bal, L-Nal, 3-(2-benzo[b]-thienyl)-L-alanine and 3-(2-naphthyl)-L-alanine.

5. A polypeptide and a pharmacologically acceptable salt thereof according to claim 1, wherein E is an amino acid selected from the group consisting of L-Tle, L-Val, L-Ile, N-Nle, L-Nva, L-Phe, L-Tle-L-α-Me-Leu, L-Pgl and L-Pgl(p-OH).

6. A polypeptide and a pharmacological acceptable salt thereof according to claim 1, wherein E is L-Tle or L-Pgl.

7. A polypeptide and a pharmacologically acceptable salt thereof according to claim 1, wherein F is an L- or D-amino acid selected from the group consisting of Gly, Ala, Val, Leu, Ile, Ser, Thr, Cys, Met, Asn, Gln, Pro, Nle, Nva, Tle and Phe.

8. A polypeptide and a pharmacologically acceptable salt thereof according to claim 1, wherein F is L-Leu.

9. A polypeptide and a pharmacologically acceptable salt thereof according to claim 1, wherein $R^1$ stands for a hydroxyl group or an alkoxy group.

10. A polypeptide and a pharmacologically acceptable salt thereof according to claim 1, wherein B is an amino acid selected from the group consisting of L-Lys, L-Arg, L-homoArg and L-Orn, C is L-Pro, D is an amino acid selected from the group consisting of L-Trp, L-Nal, L-Tyr and L-Bal, E is L-Tle or L-Pgl, F is L-Leu, and $R^1$ is a hydroxyl group or an alkoxy group.

11. A polypeptide and a pharmacologically acceptable salt thereof according to claim 10, wherein $R^1$ is a hydroxyl group.

12. A polypeptide and a pharmacologically acceptable salt thereof according to claim 10, wherein $R^1$ is a methoxy group or an ethoxy group.

13. A polypeptide and a pharmacologically acceptable salt thereof according to claim 1, wherein the compound is Gb-Arg-Pro-Tro-Pgl-Leu-OEt.

14. A polypeptide and a pharmacologically acceptable salt thereof according to claim 1, wherein the compound is Ah-Arg-Pro-Trp-Pgl-Leu-OEt.

15. A polypeptide and a pharmacologically acceptable salt thereof according to claim 1, wherein the compound is Bz-D-Arg-Lys-Pro-Trp-Tle-Leu-OEt.

16. A polypeptide and a pharmacologically acceptable salt thereof according to claim 1, wherein the compound is Bz-D-Arg-Lys-Pro-Trp-Tle-Leu-OH.

17. A polypeptide and a pharmacologically acceptable salt thereof according to claim 1, wherein the compound is H-D-Lys-Arg-Pro-Trp-Tle-Leu-OH.

18. A polypeptide and a pharmacologically acceptable salt thereof according to claim 1, wherein the compound is H(Me)Arg-Lys-Pro-Trp-Tle-Leu-OEt.

19. A polypeptide and a pharmacologically acceptable salt thereof according to claim 1, wherein the compound is H(Me)Arg-Lys-Pro-Trp-Tle-Leu-OH.

20. A polypeptide and a pharmacological acceptable salt thereof according to claim 1, wherein the compound is Ac-(Me)Arg-Lys-Pro-Trp-Tle-Leu-OH.

21. A polypeptide and a pharmacologically acceptable salt thereof according to claim 1, wherein the compound is Bz-(Me)Arg-Lys-Pro-Trp-Tle-Leu-OH.

22. A polypeptide and a pharmacologically acceptable salt thereof according to claim 1, wherein the compound is H-D-Lys-Arg-Pro-Nal-Tle-Leu-OH.

23. A polypeptide and a pharmacologically acceptable salt thereof according to claim 1, wherein the compound is HO-$CH_2$-CO-D-Arg-Lys-Pro-Trp-Tle-Leu-OH.

24. A polypeptide and a pharmacologically acceptable salt thereof according to claim 1, wherein the compound is HO-$CH_2$-CO-D-Arg-Lys-Pro-Trp-Tle-Leu-OEt.

25. A polypeptide and a pharmacologically acceptable salt thereof according to claim 1, wherein the compound is

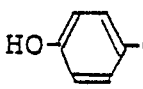-CO-D-Arg-Lys-Pro-Trp-Tle-

Leu-OH.

26. A pharmaceutical composition which comprises a therapeutically effective amount of the polypeptide as defined in Claim 1, or a pharmacologically acceptable salt thereof and a pharmacologically acceptable carrier.

27. The use of the polypeptide as defined in Claim 1 for making a medicament effective in the treatment of a mental symptom by an organic disorder in the brain.

28. The use according to Claim 27, wherein the organic disorder in the brain is a cerebrovascular disease.

29. The use according to Claim 27, wherein the organic disorder in the brain is a senile dementia.

30. The use of the polypeptide as claimed in Claim 1 for making a medicament effective in the treatment of a mental symptom associated with a cerebrovascular disease.

31. The use of the polypeptide as claimed in Claim 1 for making a medicament effective in the treatment of a mental symptom associated with a penile dementia.

32. The use of the polypeptide as defined in Claim 1 for making a medicament effective as an antipsycholotic agent.

33. The use of the polypeptide as defined in Claim 1 for making a medicament effective as an analgesic agent.

34. The use of a polypeptide as defined in Claim 1 for making a medicament effective as a methamphetamine antagonistic agent.

35. A process for producing the polypeptide or pharmacologically acceptable salt thereof as defined in Claim 1, which comprises eliminating a protected group of the polypeptide and, if necessary, conducting an acid addition reaction.

Claims for the following Contracting State: ES

1. A process for producing a polypeptide represented by the following general formula and a pharmacologically acceptable salt thereof:
A-B-C-D-E-F-$R^1$
wherein A stands for a group

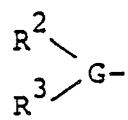

(G stands for a basic amino acid of L- or D-form, and $R^2$ and $R^3$, which may be the same or different, each stand for a hydrogen atom, a lower alkyl group or an acyl group), a group

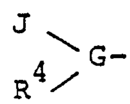

(in which G stands for a basic amino acid of L- or D-form, J stands for an amino acid of L- or D-form, and $R^4$ stands for a hydrogen atom or a lower alkyl group), an ω-aminoalkanoyl group

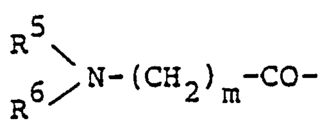

(in which $R^5$ and $R^6$, which may be the same or different, each stand for a hydrogen atom or a lower alkyl group, and m is an integer of from 1 to 10), an ω-guanidinoalkanoyl group

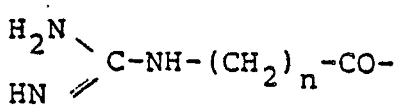

(in which n is an integer of from 1 to 10), or a group having the formula

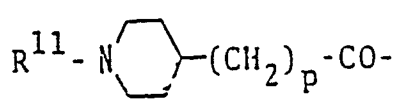

in which R11 is hydrogen or a lower alkyl, p is zero or an integer of from 1 to 10, B is a basic amino acid of L-form or an α-N-alkyl derivative thereof, C stands for L-Pro or a derivative thereof, D stands for a natural or non-natural aromatic amino acid of L-form, E stands for an amino acid of L-form or an α-N-alkyl derivative or α-C-alkyl derivative thereof, F stands for an amino acid of L- or D-form or an α-N-alkyl derivative or α-C-alkyl derivative thereof, and $R^1$ stands for a group

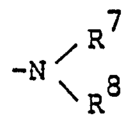

(in which $R^7$ and $R^8$, which may be the same or different, each stand for a hydrogen atom or a lower alkyl group) or a group $-O-R^9$ in which $R^9$ stands for a hydrogen atom, an alkyl group, an alkenyl group or a group $-(CH_2)_p-O-R^{10}$ in which p is an integer of from 1 to 5 and $R^{10}$ stands for an alkyl group, an alkenyl group or an acyl group),which comprises eliminating a protected group of the polypeptide and, if necessary, conducting an acid addition reaction.

2. A process according to claim 1, wherein B is an amino acid selected from the group consisting of L-Lys, L-Arg, L-homoArg and L-Orn.

3. A process according to claim 1, wherein C is L-Pro.

4. A process according to claim 1, wherein D is an amino acid selected from the group consisting of L-Tyr, L-Trp, L-Phe, L-Pgl, L-Bal, L-Nal, 3-(2-benzo[b]thienyl)-L-alanine and 3-(2-naphthyl)-L-alanine.

5. A process according to claim 1, wherein E is an amino acid selected from the group consisting of L-Tle, L-Val, L-Ile, N-Nle, L-Nva, L-Phe, L-Tle-L-α-Me-Leu, L-Pgl and L-Pgl(p-OH).

6. A process according to claim 1, wherein E is L-Tle or L-Pgl.

7. A process according to claim 1, wherein F is an L- or D-amino acid selected from the group consisting of Gly, Ala, Val, Leu, Ile, Ser, Thr, Cys, Met, Asn, Gln, Pro, Nle, Nva, Tle and Phe.

8. A process according to claim 1, wherein F is L-Leu.

9. A process according to claim 1, wherein $R^1$ stands for a hydroxyl group or an alkoxy group.

10. A process according to claim 1, wherein B is an amino acid selected from the group consisting of L-Lys, L-Arg, L-homoArg and L-Orn, C is L-Pro, D is an amino acid selected from the group consisting of L-Trp, L-Nal, L-Tyr and L-Bal, E is L-Tle or L-Pgl, F is L-Leu, and $R^1$ is a hydroxyl group or an alkoxy group.

11. A process according to claim 1, wherein $R^1$ is a hydroxyl group.

12. A process according to claim 1, wherein $R^1$ is a methoxy group or an ethoxy group.

13. A process according to claim 1, wherein the compound is Gb-Arg-Pro-Tro-Pgl-Leu-OEt.

14. A process according to claim 1, wherein the compound is Ah-Arg-Pro-Trp-Pgl-Leu-OEt.

15. A process according to claim 1, wherein the compound is Bz-D-Arg-Lys-Pro-Trp-Tle-Leu-OEt.

16. A process according to claim 1, wherein the compound is Bz-D-Arg-Lys-Pro-Trp-Tle-Leu-OH.

17. A process according to claim 1, wherein the compound is H-D-Lys-Arg-Pro-Trp-Tle-Leu-OH.

18. A process according to claim 1, wherein the compound is H(Me)Arg-Lys-Pro-Trp-Tle-Leu-OEt.

19. A process according to claim 1, wherein the compound is H(Me)Arg-Lys-Pro-Trp-Tle-Leu-OH.

20. A process according to claim 1, wherein the compound is Ac-(Me)Arg-Lys-Pro-Trp-Tle-Leu-OH.

21. A process according to claim 1, wherein the compound is Bz-(Me)Arg-Lys-Pro-Trp-Tle-Leu-OH.

22. A process according to claim 1, wherein the compound is H-D-Lys-Arg-Pro-Nal-Tle-Leu-OH.

23. A process according to claim 1, wherein the compound is $HO-CH_2-CO$-D-Arg-Lys-Pro-Trp-Tle-Leu-OH.

24. A process according to claim 1, wherein the compound is $HO-CH_2-CO$-D-Arg-Lys-Pro-Trp-Tle-Leu-OEt.

25. A process according to claim 1, wherein the compound is

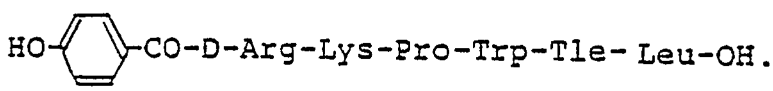

26. The use of the polypeptide as defined in Claim 1 for making a medicament effective in the treatment of a mental symptom by an organic disorder in the brain.

27. The use according to Claim 26, wherein the organic disorder in the brain is a cerebrovascular disease.

28. The use according to Claim 26, wherein the organic disorder in the brain is a senile dementia.

29. The use of the polypeptide as claimed in Claim 1 for making a medicament effective in the treatment of a mental symptom associated with a cerebrovascular disease.

30. The use of the polypeptide as claimed in Claim 1 for making a medicament effective in the treatment of a mental symptom associated with a penile dementia.

31. The use of the polypeptide as defined in Claim 1 for making a medicament effective as an antipsycholotic agent.

32. The use of the polypeptide as defined in Claim 1 for making a medicament effective as an analgesic agent.

33. The use of a polypeptide as defined in Claim 1 for making a medicament effective as a methamphetamine antagonistic agent.